# EUROPEAN PATENT SPECIFICATION

(11) **EP 4 141 882 B1**
(45) Date of publication and mention of the grant of the patent: **18.03.2026**
(21) Application number: 22190359.4
(22) Date of filing: 15.08.2022
(51) Int. Cl.: G16H 20/30, G16H 50/30

(54) **SYSTEMS, APPARATUS, AND METHODS FOR MUSCULOSKELETAL ERGONOMIC IMPROVEMENT**
SYSTEME, GERÄTE UND VERFAHREN ZUR MUSKOLOSKELETTALEN ERGONOMISCHEN VERBESSERUNG
SYSTÈMES, APPAREIL ET PROCÉDÉS D'AMÉLIORATION ERGONOMIQUE MUSCULOSQUELETTIQUE

(30) Priority: 25.08.2021 US 202117412038
(43) Date of publication of application: 01.03.2023
(73) Proprietor: The Boeing Company, Arlington, VA 22202 (US)
(72) Inventor: LAUGHLIN, Brian D, Chicago, 60606-2016 (US); GEORGESON, Gary E, Chicago, 60606-2016 (US)
(74) Representative: Boult Wade Tennant LLP

(56) References cited:
- CN-A- 109 984 747
- US-B2- 10 838 373

## Description

### FIELD OF THE DISCLOSURE

This disclosure relates generally to ergonomics and, more particularly, to systems, apparatus, and methods for musculoskeletal ergonomic improvement.

### BACKGROUND

An individual may experience a musculoskeletal injury (e.g., an injury to muscle(s), nerve(s), and/or joint(s) of the individual's body) while performing activities. Such injuries can stem from conditions in a work environment and/or a manner in which the activities are performed.

US 10838373, in accordance with its abstract, states that in automatic operation of an ergonomic equipment according to dynamic workplace conditions, an activity being performed in a workplace by a person at a first time is determined based on an input from a data processing system. A group is selected corresponding to the activity. An ergonomic policy is modified to form a modified ergonomic policy. The ergonomic policy applies to the activity, and the modification is based on a condition existing in the workplace at the first time of the person performing the activity. Independent of a participation of the person, and when the person is not in compliance with the modified ergonomic policy, an ergonomic control is activated. The ergonomic control causes a change in an ergonomic feature of the ergonomic equipment, the change causing the person to become compliant with the modified ergonomic policy.

CN 109984747, in accordance with its abstract, states a comprehensive assessment instrument for a fall-down risk. The instrument comprises a large-pressure sensing felt, a front photographing instrument with a lens with a horizontal ruler, a testing table and a rear photographing instrument with a lens with a horizontal ruler. One end of the testing table is parallelly provided with a left column mounting groove, a middle column mounting groove and a right column mounting groove, the other end of the testing table is parallelly provided with a left gravity transmission instrument weighing table and a right gravity transmission instrument weighing table, an iron chain hook is installed on the portion, between the left gravity transmission instrument weighing table and the right gravity transmission instrument weighing table, of the testing table, and foot pressure sensing felts are installed at the tops of the left gravity transmission instrument weighing table and the right gravity transmission instrument weighing table.

### SUMMARY

There is described here, an apparatus comprising: a user profile generator configured to generate a user profile for a user based on strain sensor data collected via one or more strain sensors associated with the user, the user profile including the strain sensor data, historical sensor data for the user, and user parameter data; an aggregator configured to aggregate the strain sensor data for the user with strain sensor data for a population of users to generate a population profile comprising population data; a performance analyzer to determine a likelihood of the user experiencing a musculoskeletal strain event at a portion of the body of the user based on the strain sensor data and by comparing the user parameter data stored in the user profile to the population profile, wherein the performance analyzer predicts that the user is at risk of a musculoskeletal strain event if the strain sensor data indicates that the portion of the body of the user is under a strain which exceeds an amount associated with an average or an optimal exertion by the user based on the comparison of the user parameter data and the population; and transmit, in response to the prediction of the strain event, an instruction including an alert to be output by the output device; and an ergonomic form recommendation generator to transmit, in response to the prediction of the strain event, an instruction including an ergonomic form measure to be output by the output device, wherein the ergonomic form measure is generated based on the user profile.

There is also described herein, a method comprising: generating a user profile for a user based on strain sensor data collected via one or more strain sensors associated with the user, the user profile including the strain sensor data, historical sensor data for the user, and user parameter data; aggregating the strain sensor data for the user with strain sensor data for a population of users to generate a population profile comprising population data; determining a likelihood of the user experiencing a musculoskeletal strain event at a portion of the body of the user based on the strain sensor data and by comparing the user parameter data stored in the user profile to the population profile; predicting that the user is at risk of a musculoskeletal strain event if the strain sensor data indicates that portion of the body of the user is under a strain which exceeds an amount associated with an average or an optimal exertion by the user based on the comparison of the user parameter data and the population data; and transmitting, in response to the prediction of the musculoskeletal strain event, an instruction including an alert and an ergonomic form measure to be output by an output device, wherein the ergonomic form measure is generated based on the user profile.

There is also described herein, a computer program comprising computer program instructions that, when executed by a computer processor, cause the computer processor to execute a method comprising: generating a user profile for a user based on strain sensor data collected via one or more strain sensors associated with the user, the user profile including the strain sensor data, historical sensor data for the user, and user parameter data; aggregating the strain sensor data for the user with strain sensor data for a population of users to generate a population profile comprising population data; determining a likelihood of the user experiencing a musculoskeletal strain event at a portion of the body of the user based on the strain sensor data and by comparing the user parameter data stored in the user profile to the population profile; predicting that the user is at risk of a musculoskeletal strain event if the strain sensor data indicates that portion of the body of the user is under a strain which exceeds an amount associated with an average or an optimal exertion by the user based on the comparison of the user parameter data and the population data; and transmitting, in response to the prediction of the musculoskeletal strain event, an instruction including an alert and an ergonomic form measure to be output by an output device, wherein the ergonomic form measure is generated based on the user profile, or a non-transitory computer-readable medium having stored thereon such a computer program.

### BRIEF DESCRIPTION OF THE DRAWINGS

FIG. 1 illustrates an example system constructed in accordance with teachings of this disclosure and including data collection device(s) for collecting biological, ergonomic, and/or environmental data associated with a user and an ergonomic analysis controller for predicting a likelihood of the user experiencing a musculoskeletal strain event.
FIG. 2 is a block diagram of the example system of FIG. 1 including an example implementation of the ergonomic analysis controller of FIG. 1, an aggregator to generate population data profile(s), and one or more computing system for training neural network(s) to generate model(s) for by the ergonomic analysis controller in evaluating ergonomic form of the user.
FIG. 3 is a flowchart representative of example machine readable instructions that, when executed by the example aggregator of FIGS. 1 and/or 2, causes the aggregator to generate population data profile(s) for two or more users.
FIG. 4 is a flowchart representative of example machine readable instructions that, when executed by a first computing system of the example system of FIG. 2, cause the first computing system to train a neural network to predict a likelihood of a musculoskeletal strain event.
FIG. 5 is a flowchart representative of example machine readable instructions that, when executed by the ergonomic analysis controller of FIGS. 1 and/or 2, cause the ergonomic analysis controller to predict a likelihood of a musculoskeletal strain event.
FIG. 6 is a block diagram of an example processing platform structured to execute the instructions of FIG. 3 to implement the example aggregator of FIGS. 1 and/or 2.
FIG. 7 is a block diagram of an example processing platform structured to execute the instructions of FIG. 4 to implement the example first computing system of FIG. 2.
FIG. 8 is a block diagram of an example processing platform structured to execute the instructions of FIG. 5 to implement the example ergonomic analysis controller of FIGS. 1 and/or 2.

The figures are not to scale. In general, the same reference numbers will be used throughout the drawing(s) and accompanying written description to refer to the same or like parts.

Unless specifically stated otherwise, descriptors such as "first," "second," "third," etc. are used herein without imputing or otherwise indicating any meaning of priority, physical order, arrangement in a list, and/or ordering in any way, but are merely used as labels and/or arbitrary names to distinguish elements for ease of understanding the disclosed examples. In some examples, the descriptor "first" may be used to refer to an element in the detailed description, while the same element may be referred to in a claim with a different descriptor such as "second" or "third." In such instances, it should be understood that such descriptors are used merely for identifying those elements distinctly that might, for example, otherwise share a same name.

### DETAILED DESCRIPTION

An individual may experience a musculoskeletal injury (e.g., an injury to muscle(s), nerve(s), and/or joint(s) of the individual's body) while performing activities. Such injuries can stem from conditions in a work environment and/or a manner in which the activities are performed. For instance, performing repetitive tasks, lifting heavy objects, and/or other types of overuse or overexertion activities can cause musculoskeletal injuries that, in addition to causing pain, may affect worker productivity. Workplace conditions such as a layout of a workspace and/or a design of objects in the workspace, such as a height of a desk, can contribute to musculoskeletal injuries in an individual over time. In some examples, repeated exposure to conditions in the environment such as vibrations can also cause musculoskeletal injuries. Efforts to reduce musculoskeletal injuries are often not addressed until the worker is experiencing pain.

Disclosed herein are example systems, apparatus, and methods that predict a likelihood that a user is experiencing or is likely to experience a musculoskeletal strain event or is likely to experience a musculoskeletal strain event to notify the user of the predicted strain event. Some examples disclosed herein access data generated by one or more sensors associated with the user and/or located in the environment in which the user is performing movements. The sensors can include wearable sensors (e.g., biosensors to detect body temperature, heart rate, hydration level, etc.; strain sensors carried by a fabric worn by the user that detect muscle strain and/or tension). In some examples, the sensors include environmental sensors such as video cameras to capture images of the user in the environment and/or infrared or thermal cameras to detect heat generated by the user.

In some examples disclosed herein, an ergonomic analysis controller executes neural network model(s) to evaluate ergonomic form(s) associated with a user's body and to identify a risk of strain event(s) for one or more portions of the user's body based on the sensor data. The neural network model(s) can be generated for detecting strain event(s) at particular portions of the user's body, such as a shoulder. In some examples disclosed herein, the ergonomic analysis controller analyzes the results of the neural network analysis in view of data previously collected from the user and/or other users to identify trends in user movement that can indicate that the user is overstressing one or more portions of his or her body (e.g., to detect repetitive motion or to identify anomalies in user movement that can lead to injury). In some examples disclosed herein, data collected from the user and/or other users is used to refine the neural network model(s) and, thus, the predictions of musculoskeletal strain event(s).

Some examples disclosed herein provide feedback to the user to alert the user to the predicted strain event. The alert(s) can be provided via one or more output devices, such as via a user application on a smartphone and/or wearable device such as a smartwatch. Some examples disclosed herein provide recommendations or mitigation instructions as to how the user can alleviate strain and/or otherwise improve ergonomic form. The recommendations can include, for instance, audio instructions and/or visual instructions advising the user how to perform a movement safely, recommendations as to the number or repetitions of a movement to perform, etc. Some examples disclosed herein dynamically respond to changes in user characteristics and/or behavior in evaluating the risk for strain event(s) and/or developing ergonomic form recommendations, rather than relying on static reference data that may or may not be accurate for the user.

In some examples disclosed herein, the sensor data and/or results of analysis of the sensor data performed by the ergonomic analysis controller for the user and/or a population of users are provided to third parties such as a healthcare provider. Such information can be used by healthcare providers to, for instance, monitor the user(s) and develop a customized health plan to reduce the risk of musculoskeletal injuries.

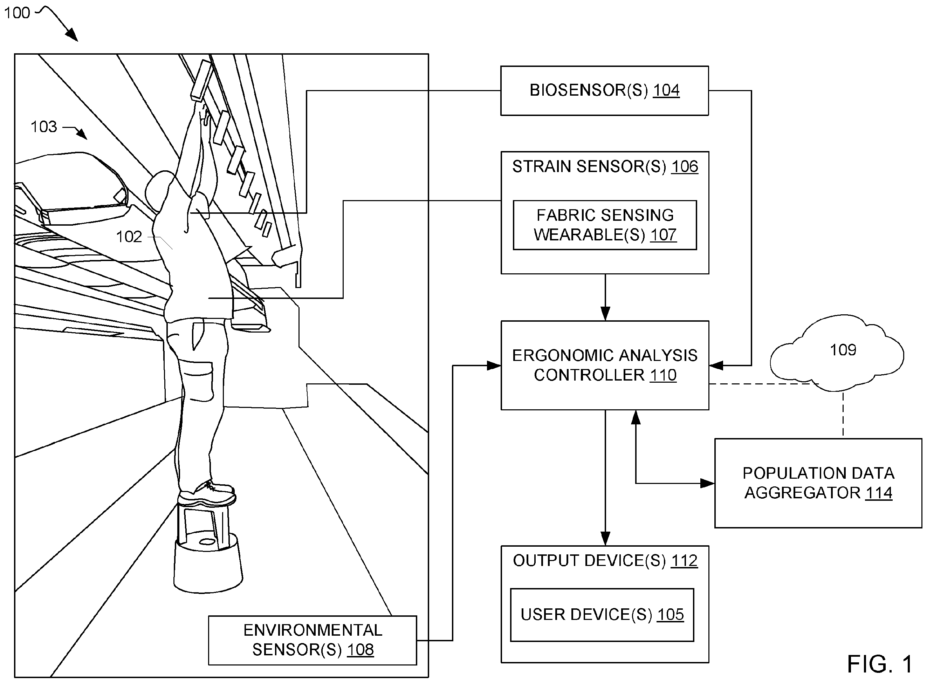

FIG. 1 illustrates an example system 100 constructed in accordance with teachings of this disclosure for predicting a likelihood of musculoskeletal strain event(s) for a user 102 performing movement(s) in an environment 103. As shown in FIG. 1, the user 102 (the terms "user" and "subject" are used interchangeably herein and both refer to a human being) is performing an activity in the environment 103 that involves the user 102 raising his or her arms over his or her head. For instance, in the example of FIG. 1, the user 102 is installing components of an aircraft. The user 102 may perform other motions, activities, etc. than the example overhead position shown in FIG. 1. Also, the environment 103 can differ from the example shown in FIG. 1.

The example system 100 includes one or more sensors to collect biological data from the user 102. For example, the sensor(s) can include biosensor(s) 104 carried by the user 102 to collect biological data for the user 102 such as heart rate, respiration rate, blood pressure, body temperature, hydration level, etc. In some examples, the biosensor(s) 104 are carried by one or more user devices 105, such as a smartwatch or a health tracker. The user 102 may carry (e.g., wear) the user device(s) 105 to enable the biosensor(s) 104 of the user device(s) 105 to collect data from the user 102.

The example system 100 includes one or more strain sensor(s) 106 to detect strain and/or stress on joint(s) of the user 102 and/or with respect to the muscle(s) of the user 102. The strain sensor(s) 106 can include electromyography (EMG) sensor(s) worn by the user 102 to detect muscle tension. In some examples, the strain sensor(s) 106 include sensor(s) to detect skin and/or muscle temperature, which are indicative of muscle activity. In some examples, other types of sensors, such as position sensors and/or accelerometers are carried by the user 102 and/or by user device(s) 105 associated with the user 102 to output data indicative of muscle strain.

In some examples, the strain sensor(s) 106 include fabric sensing wearable(s) 107. The fabric sensing wearable(s) 107 include wearable fabrics (e.g., a shirt or other garment) that include sensor(s) to output data indicative of strain on the muscle(s) and/or skeleton (e.g., joint(s)) of the user 102. For example, motion-sensing fabrics can include pressure and/or strain sensor(s) that output signal(s) in response to changes in pressure and/or deformation of the sensor(s) during movement by the user 102.

In some examples, the system 100 includes environmental sensor(s) 108, or sensor(s) located in the environment 103, that collect data with respect to the environment 103 and/or the user 102 in the environment 103. The environmental sensor(s) 108 can include, for example, camera(s) (e.g., video camera(s), still camera(s)) to generate image data of the user 102 in the environment 103, audio sensor(s) to capture audio in the environment 103, vibration sensor(s) to detect vibrations in the environment 103, motion capture sensor(s), etc. In some examples, the environmental sensor(s) 108 include infrared camera(s) that detect changes in a temperature of a skin of the user 102 due to muscle activity.

The example system 100 can include other types of sensors than the example sensors 104, 106, 107, 108 disclosed herein. Also, in some examples, the system 100 includes fewer types of sensor(s). For example, the system 100 can include the biosensor(s) 104 and/or the strain sensor(s) 106 but not the environmental sensor(s) 108.

In the example of FIG. 1, the signals output by the biosensor(s) 104, the strain sensor(s) 106 (including the fabric sensing wearable(s) 107), and/or the environmental sensor(s) 108 are transmitted to an ergonomic analysis controller 110. In the example of FIG. 1, the ergonomic analysis controller 110 is implemented by one or more cloud-based device(s) 109 such as one or more servers, processors, and/or virtual machines. In other examples, some of the analysis performed by the ergonomic analysis controller 110 is implemented by the cloud-based device(s) 109 and other parts of the analysis are implemented by processor(s) of one or more user devices 105 (e.g., a smartphone, a personal computing device such as an electronic tablet or laptop).

In the example of FIG. 1, the biosensor(s) 104, the strain sensor(s) 106, and the environmental sensor(s) 108 are in communication with the ergonomic analysis controller 110 via wireless connection(s) (e.g., Bluetooth^{®}, WiFi connections with the sensor(s) 104, 106, 108 and/or with the user device(s) 105 carrying the sensor(s) 104, 106, 108). In some examples, the ergonomic analysis controller 110 receives sensor data from sensor(s) 104, 106, 108 in real-time or substantially real-time (as used herein "real-time" and "substantially real time" refers to occurrence in a near instantaneous manner (e.g., +/- 1 second) recognizing there may be real world delays for computing time, transmission, etc.). In other examples, the ergonomic analysis controller 110 receives the sensor data at a later time (e.g., periodically and/or aperiodically based on one or more settings but sometime after the activity that caused the sensor data to be generated, such as movement by the user 102, has occurred (e.g., seconds later)). The ergonomic analysis controller 110 can perform one or more operations on the sensor data such as filtering the raw signal data, removing noise from the signal data, and/or converting the signal data from analog to digital data.

The example ergonomic analysis controller 110 analyzes the sensor signal data from the respective sensor(s) 104, 106, 108 to predict a likelihood that one or more portions of the body of the user 102 is under strain such that there is a risk of comprising musculoskeletal integrity. As disclosed herein, the ergonomic analysis controller 110 implements neural network model(s) to predict if the one or more portions of the user's body (e.g., muscle(s), joint(s)) is experiencing a musculoskeletal strain event or is likely to experience a strain event. The neural network model(s) can be trained using previously collected data (e.g., biometric sensor data, image data, reference anthropometric data) associated with the user and/or other individuals. The training data can define baseline or threshold information for determining if the user is at risk for experiencing a musculoskeletal strain event. In such examples, the ergonomic analysis controller 110 predicts the musculoskeletal strain event(s) by executing the trained neural network model(s) for the sensor signal data generated by the sensor(s) 104, 106, 108. In some examples, the ergonomic analysis controller 110 determines that the user 102 is experiencing a musculoskeletal strain event or is likely to experience a strain event by mapping one or more user parameters (e.g., gender, age, weight, athletic ability) to population profile data. The population profile data can include, for example, average ranges of motion for users based on parameters such as weight, gender, athletic ability; average weight that can be safely lifted based on age, gender, etc.

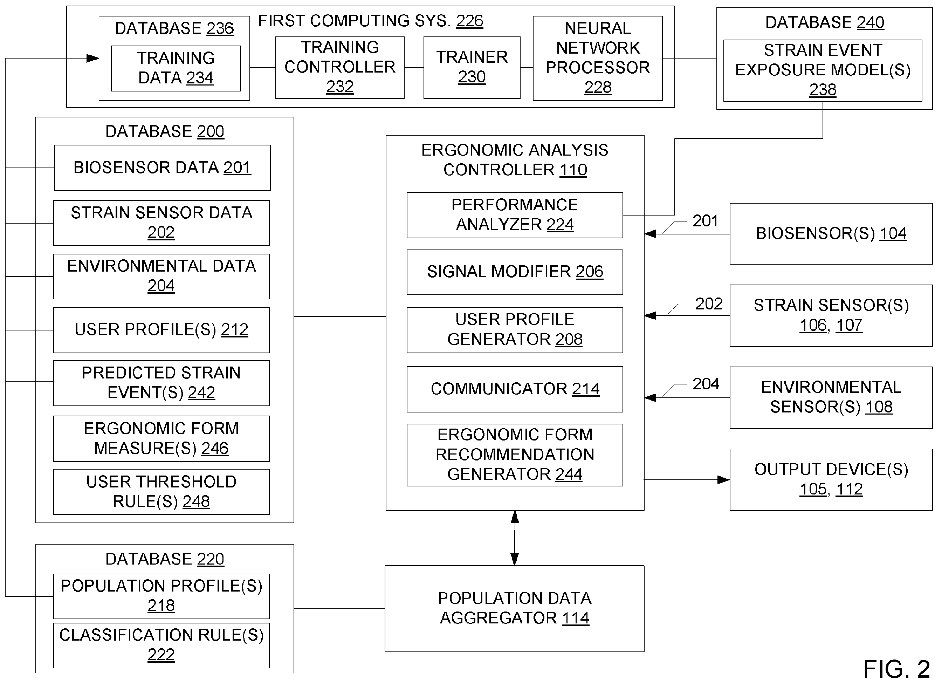

In some examples disclosed herein, the ergonomic analysis controller 110 predicts the strain event(s) based on the signal data generated by the sensor(s) 104, 106, 108 while the user 102 is in the environment 103 and/or data previously collected from the user (in the environment 103 and/or in other environment(s)). The previously collected data can include biosensor data and/or strain sensor data and can serve as baseline or reference data for the user 102. In some examples, previously collected data from the environment 103 by the environmental sensor(s) 108 (e.g., vibration levels) and/or previously collected sensor data from other environments similar to the environment 103 (e.g., manufacturing environments) serves as reference environmental data. As disclosed herein (FIG. 2), the reference data can be stored in one or more databases (e.g., cloud-based storage device(s)) and accessed by the ergonomic analysis controller 110. In some examples, the reference data is updated in response to new data received from the user 102, other user(s), the environment 103, and/or other environment(s).

In some examples, the ergonomic analysis controller 110 compares the results of the neural network analysis in view of previously collected sensor data for the user 102 and/or previously generated neural network analysis results for the user 102 to verify that a prediction that the user 102 is or is not likely to experience a strain event is accurate. For example, for a given set of sensor data, the ergonomic analysis controller 110 may determine that the user 102 is not likely to experience a strain event. However, based on previously generated neural network results and/or historical sensor data for the user 102, the ergonomic analysis controller 110 may determine that the user 102 is at risk for a strain event due to the cumulative effect of strain from, for instance, performing a repetitive motion. Thus, in some examples, the ergonomic analysis controller 110 predicts that the user 102 is experiencing or is likely to experience a musculoskeletal strain event based on cumulative results from the neural network analysis and/or changes in the sensor data collected from the user 102 and/or the environment 103 over time. The neural network model(s) implemented by the ergonomic analysis controller 110 of FIG. 1 are refined and/or updated based on the data collected from the user 102 and/or for the environment 103 (or other environment(s) from which data is collected from the user 102) over time and stored in one or more databases (e.g., the database 200 of FIG. 2). Thus, in the example of FIG. 1, the prediction of the strain event(s) is customized for the user 102 in view of data and/or trends for the user 102 over time.

In the example of FIG. 1, the ergonomic analysis controller 110 is in communication with one or more output device(s) 112 to provide alert(s) to the user 102 in response to prediction(s) by the ergonomic analysis controller 110 that the user 102 is likely to experience strain event(s). The output device(s) 112 can include, for example, user device(s) 105 (e.g., smartphones, electronic tablets and the like) associated with the user 102, including wearable device(s) (e.g., smartwatches) worn by the user 102. In some examples, the output device(s) 112 are the same device(s) that include the biosensor(s) 104, the strain sensor(s) 106, and/or the environmental sensor(s) 108. The alert(s) can include, for instance, audio alert(s), visual alert(s), and/or haptic feedback alert(s). For example, a visual alert can be displayed via a user interface of a user application installed on a smartphone. The ergonomic analysis controller 110 can instruct the output device(s) 112 to output the alert(s) in response to prediction(s) by the ergonomic analysis controller 110 that movement(s) by the user 102 and/or cumulative movement(s) by the user 102 over time are associated with strain event(s) that can result injury to the user 102.

In some examples, the output device(s) 112 include user device(s) (e.g., electronic tablets, smartphones, laptops) associated with a third party who is authorized to receive report(s), alert(s), etc. with respect to the analysis of the sensor data and/or prediction(s) of strain event(s). The third party can include, for example, a medical professional. In some examples, the ergonomic analysis controller 110 transmits the data collected by the sensor(s) 104, 106, 108 and/or data derived therefrom (e.g., average muscle strain data) for display at the output device(s) 112. Thus, the authorized third party can track changes in the user 102 with respect to musculoskeletal events over time.

In some examples, the ergonomic analysis controller 110 generates ergonomic form recommendation(s) for the user 102 in response to predicting a likelihood that the user 102 is experiencing strain event(s). For example, the ergonomic form recommendation(s) can include instruction(s) or action(s) that the user 102 can take to alleviate stress or strain on the portion(s) of the user's body (e.g., by re-positioning the user's body part, taking a break from the movement, etc.). The ergonomic form recommendation(s) can include, for instance, recommended limits on a number of repetitions of a movement performed by the user 102, recommended limits on an amount of weight that the user 102 can safely carry, etc. As disclosed herein, the ergonomic form recommendation(s) can be generated based on the data collected from the user 102 via the sensor(s) 104, 106, 108 over time and predefined ergonomic form rule(s). The ergonomic form recommendation(s) generated by the ergonomic analysis controller 110 can include, for instance, visual instruction(s) that are displayed via a display screen of the output device(s) 112 and/or audio instruction(s) that are presented via speaker(s) of the output device(s) 112, etc.

As disclosed herein, the neural network analysis with respect to musculoskeletal strain event(s) and/or the generation of the ergonomic form recommendation(s) can be based on sensor data collected from the user 102 for whom the analysis is performed, including sensor data collected from the user 102 over time. In some examples, the ergonomic analysis controller 110 also uses data collected from other users to refine the neural network analysis and/or generate the ergonomic form recommendation(s). Thus, in some examples, the ergonomic analysis controller 110 performs a population-based analysis of strain event(s) associated with the user 102.

In the example system 100 of FIG. 1, the ergonomic analysis controller 110 is in communication with a population data aggregator 114. In some examples, the ergonomic analysis controller 110 incudes the population data aggregator 114. In other examples, the population data aggregator 114 is implemented by one or more cloud-based device(s) such as one or more servers, processors, and/or virtual machines and/or one or more user devices (e.g., smartphone, a personal computing device such as an electronic tablet or laptop) different from the cloud-based device(s) and/or user device(s) that implement the ergonomic analysis controller 110.

The example population data aggregator 114 of FIG. 1 provides means for aggregating data associated with two or more users to generate population-based data for the ergonomic form analysis performed by the ergonomic analysis controller 110. In some examples, the ergonomic analysis controller 110 transmits data from the sensor(s) 104, 106, 108 associated with the user 102 to the population data aggregator 114 for aggregation with data from other users. Also, in some examples, the ergonomic analysis controller 110 transmits the results of the neural network analysis with respect to the predicted strain event(s) for the user 102 based on the analysis of the sensor data from the sensor(s) 104, 106, 108, to the population data aggregator 114. In other examples, the sensor(s) 104, 106, 108 transmit the data for the user 102 directly to the population data aggregator 114 and the ergonomic analysis controller 110 retrieve the data from the population data aggregator 114. The ergonomic analysis controller 110 communicates with the population data aggregator 114 via one or more wireless connections.

The example population data aggregator 114 receives data associated with the user 102 and other users. For instance, the population data aggregator 114 can receive biosensor data collected from other users in response to the other users performing movements in the environment 103 and/or different environments. In some examples, data is collected from the other users in response to the users performing the same or substantially the same movements as performed by the user 102 (e.g., an overhead movement). Additionally or alternatively, the data can be collected from the other users in response to the users performing different movements than the user 102.

The example population data aggregator 114 classifies or groups the data associated with the plurality of users based on variables such as individual characteristics (e.g., age, gender, etc.), movement types, and/or environment(s) from which the data was collected. As a result, the population data aggregator 114 generates population profile data including data profiles defined by different classifications (e.g., demographics, environment type, movement type). The classifications defined by the population data aggregator 114 can be customized based on, for instance, properties of the environment 103 (e.g., type of work performed) and/or reference data such as anthropometric measurements for individuals of different ages, genders, etc.

In some examples, the population data aggregator 114 aggregates data from individuals in the population over time and determines average or threshold data for detecting strain event(s) based on the data collected from the population over time. For instance, the population data profile(s) can define averages of, for instance, biosensor data (e.g., heart rate data) and/or strain sensor data (e.g., amount of muscle strain or tension detected) from multiple users who experienced musculoskeletal injury. In the example of FIG. 1, the population data aggregator 114 updates the population-based profile data as new data is received for users.

In some examples of FIG. 1, the population-based data generated by the population data aggregator 114 is used to train the neural network model(s) executed by the ergonomic analysis controller 110. In some examples, particular population-based data is selected for training the neural network model(s) based on a body part of interest (e.g., a shoulder), properties of user(s) (e.g., demographics), environment type, and/or the type of sensor data collected from the user(s). In some examples, the ergonomic analysis controller 110 compares the sensor data for the user 102 to the population-based data to determine whether the user 102 is at risk for strain event(s) in view of the trends in a larger population size (e.g., when verifying the predicted strain event(s)).

FIG. 2 is a block diagram of an example implementation of the ergonomic analysis controller 110 of FIG. 1. As mentioned above, the example ergonomic analysis controller 110 is constructed to predict a likelihood of musculoskeletal strain event(s) to one or more portions of a body of a user (e.g., the user 102 of FIG. 1) based on sensor data collected from or associated with the user in response to movement(s) performed by the user in an environment (e.g., the environment 103 of FIG. 1). In the example of FIG. 2, the ergonomic analysis controller 110 is implemented by one or more processor(s) of user device(s) (e.g., the user device(s) 105, 112 of FIG. 1) and/or cloud-based device(s) (e.g., the cloud-based device(s) 109 of FIG. 1 including server(s), processor(s), and/or virtual machine(s)). In some examples, some of the analysis is implemented by the ergonomic analysis controller 110 via a cloud-computing environment and one or more other parts of the analysis is implemented by processor(s) of one or more user device(s).

As also disclosed herein, the example population data aggregator 114 is constructed to aggregate or compile data associated with a plurality of users (including, for example, the user 102 of FIG. 1). In the example of FIG. 2, the population data aggregator 114 is implemented by one or more processor(s) of user device(s) (e.g., the user device(s) 105, 112 of FIG. 1) and/or cloud-based device(s) (e.g., the cloud-based device(s) 109 of FIG. 1 including server(s), processor(s), and/or virtual machine(s)). In some examples, some of the analysis is implemented by the population data aggregator 114 via a cloud-computing environment and one or more other parts of the analysis is implemented by processor(s) of one or more user device(s). In some examples, the ergonomic analysis controller 110 includes the population data aggregator 114. In some examples, one or more components of the ergonomic analysis controller 110 and the population data aggregator 114 are implemented by the same cloud-based device(s) and/or user device(s).

In the example of FIG. 2, biosensor data 201 collected from the user 102 of FIG. 1 via the biosensor(s) 104; strain sensor data 202 collected from the user 102 via the strain sensor(s) 106, 107; and/or environmental data 204 (e.g., image(s) of the user 102 in the environment 103 of FIG. 1) collected via the environmental sensor(s) 108 when the user 102 is in the environment 103 is transmitted to the ergonomic analysis controller 110. This transmission may be in real time/substantially in real time (e.g., as the data is gathered), periodically (e.g., every five seconds), and/or may be aperiodic (e.g., based on factor(s) such as an amount of data collected, memory storage capacity usage, whether the user 102 has performed a movement, etc.).

In the example of FIG. 2, a database 200 provides means for storing the biosensor data 201, the strain sensor data 202, and the environmental data 204. In the example of FIG. 2, the database 200 stores the data 201, 202, 204 as the data 201, 202, 204 is received by the ergonomic analysis controller 110 over time. In some examples, the database 200 stores the data 201, 202, 204 based on variables such as the environment 103 from which the data 201, 202, 204 was collected, a time at which the data 201, 202, 204 was collected, a type of movement performed by the user 102, etc. Although examples disclosed herein generally refer to the sensor data 201, 202, 204, in some examples, not all types of the sensor data 201, 202, 204 is collected and/or used in the analyses performed herein.

In some examples, the ergonomic analysis controller 110 includes the database 200. In other examples, the database 200 is located external to the ergonomic analysis controller 110 in a location accessible to the ergonomic analysis controller 110 as shown in FIG. 2.

The example ergonomic analysis controller 110 includes a signal modifier 206. The signal modifier 206 can perform operations to modify the sensor data 201, 202, 204 from the sensor(s) 104, 106, 107, 108 to, for example, filter the data, convert time domain audio data into the frequency spectrum (e.g., via Fast Fourier Transform (FFT) processing) for spectral analysis, etc. In some examples, the data 201, 202, 204 undergoes modification(s) by the signal modifier 206 before being stored in the database 200.

The example ergonomic analysis controller 110 of FIG. 2 includes a user profile generator 208. The user profile generator 208 of FIG. 2 analyzes the biosensor data 201, the strain sensor data 202, and/or the environmental data 204 to identify characteristic(s) of the user 102 and to track the user characteristic(s) over time. For example, the user profile generator 208 can determine average or baseline metrics for the user 102 such as a heartrate, blood pressure, body temperature, etc. based on the biosensor data 201 collected by the biosensor(s) 104. The user profile generator 208 can determine average or baseline metrics for the user 102 with respect to muscle strain, tension, and/or intensity based on the strain sensor data 202 from the strain sensor(s) 106, 107. The user profile generator 208 can perform image recognition analysis on image data of the user 102 captured by the environmental sensor(s) 108 to recognize movement(s) performed by the user 102.

The user profile generator 208 generates one or more user profile(s) 212 for the user 102 based on the analysis of the sensor data 201, 202, 204. For example, the user profile generator 208 can generate a first user profile 212 including heart rate data for the user 102 collected over time. The user profile generator 208 can generate a second user profile 212 including muscle tension detected by the strain sensor(s) 106, 107 during movement of one or more portion(s) of the body of the user 102 over time. The example user profile generator 208 of FIG. 2 updates the user profile(s) 212 or generates new user profile(s) 212 in response to additional data 201, 202, 204 collected by the sensors 104, 106, 107, 108. The user profile(s) 212 can include other data such as an age, gender, race, medical condition(s), athletic ability, etc. The user profile(s) 212 are stored in the database 200 and can serve as reference or historical data for the user 102.

The example ergonomic analysis controller 110 of FIG. 2 includes a communicator 214. In the example of FIG. 2, the communicator 214 provides means for communicating with the population data aggregator 114 to cause one or more of the biosensor data 201, the strain sensor data 202, the environmental data 204, and/or the user profile(s) 212 to be transmitted to the population data aggregator 114 for compilation with data associated with other users.

In the example of FIG. 2, the population data aggregator 114 aggregates or complies the sensor data 201, 202, 204 and/or the user profile(s) 212 with sensor data and/or profiles associated with other users. In particular, the population data aggregator 114 generates population profile(s) 218 that include data associated with the user 102 and other users classified based on variables such as user demographics (e.g., age, gender), sensor data type (e.g., heart sensor data, strain sensor data), movement type, environment type, etc. and saved as respective data profiles. The classifications can be defined by population data classification rule(s) 222. The population data classification rule(s) 222 can define classification(s) for grouping the sensor data associated with different user(s). The classification(s) can be defined based on user input(s). In the example of FIG. 2, the population profile(s) 218 and the population data classification rule(s) are stored in a database 220. In some examples, the population data aggregator 114 includes the database 220. In other examples, the database 220 is located external to the population data aggregator 114 in a location accessible to the population data aggregator 114 as shown in FIG. 1. In some examples, the databases 200, 220 are the same database.

In the example of FIG. 2, the population data aggregator 114 updates the population profile(s) 218 as additional data associated with user(s) and/or environment(s) is received by the population data aggregator 114. In some examples, the population data aggregator 114 receives sensor data and/or user profile data for individual user(s) periodically and/or aperiodically (e.g., based on factor(s) such as an amount of data to be transmitted to the population data aggregator 114, memory storage capacity usage, etc.).

In some examples, the database 220 of the population data aggregator 114 stores individual user profiles and/or sensor data associated with respective users (e.g., the user 102 of FIG. 1) in addition to the population profile(s) 218. For example, the sensor data 201, 202, 204 and/or the user profile(s) 212 associated with the user 102 of FIG. 1 can be stored at the database 220 associated with the population data aggregator 114 instead of the database 200 of the ergonomic analysis controller 110. In some examples, the data 201, 202, 204 is initially stored in the database 200 and then removed from the database 200 after the data 201, 202, 204 is provided to the population data aggregator 114.

The example ergonomic analysis controller 110 of FIG. 2 includes a performance analyzer 224. In some examples, the performance analyzer 224 determines a likelihood of the user 102 experiencing a musculoskeletal strain event at one or more portions of the body of the user 102 based on the sensor data 201, 202, 204 and by comparing user parameter data (e.g., one or more characteristics of the user 102 such as age, gender, race, weight, athletic ability, medical condition(s), etc., which can be stored in the user profile(s) 212) to the population profile(s) 218. For example, the performance analyzer 224 can map parameters associated with a first user who is a male having an age of 55 and weighing 200 pounds with population data for other individuals having similar parameters and an average weight that can be lifted by such individuals without comprising ergonomic form, subjecting the user's muscle and/or joints to excessive stress, etc. The population analyzer 224 can determine that the first user is at risk of a strain event based on the population-based comparison if the strain sensor data 201 indicates that one or more portions of the body of the first user 102 is under strain exceeds an amount associated with average, or optimal exertion by the first user based on the first user's parameters and the population data.

In some examples, the performance analyzer 224 executes neural network model(s) to determine a likelihood of the user 102 experiencing a musculoskeletal strain event, or a musculoskeletal event to one or more portions of the body of the user 102.

Artificial intelligence (AI), including machine learning (ML), deep learning (DL), and/or other artificial machine-driven logic, enables machines (e.g., computers, logic circuits, etc.) to use a model to process input data to generate an output based on patterns and/or associations previously learned by the model via a training process. For instance, the model may be trained with data to recognize patterns and/or associations and follow such patterns and/or associations when processing input data such that other input(s) result in output(s) consistent with the recognized patterns and/or associations.

In general, implementing a ML/AI system involves two phases, a learning/training phase and an inference phase. In the learning/training phase, a training algorithm is used to train a model to operate in accordance with patterns and/or associations based on, for example, training data. In general, the model includes internal parameters that guide how input data is transformed into output data, such as through a series of nodes and connections within the model to transform input data into output data. Additionally, hyperparameters are used as part of the training process to control how the learning is performed (e.g., a learning rate, a number of layers to be used in the machine learning model, etc.). Hyperparameters are defined to be training parameters that are determined prior to initiating the training process.

Different types of training may be performed based on the type of ML/AI model and/or the expected output. For example, supervised training uses inputs and corresponding expected (e.g., labeled) outputs to select parameters (e.g., by iterating over combinations of select parameters) for the ML/AI model that reduce model error. As used herein, labelling refers to an expected output of the machine learning model (e.g., a classification, an expected output value, etc.). Alternatively, unsupervised training (e.g., used in deep learning, a subset of machine learning, etc.) involves inferring patterns from inputs to select parameters for the ML/AI model (e.g., without the benefit of expected (e.g., labeled) outputs).

Training is performed using training data. In examples disclosed herein, the training data originates from previously generated sensor data (e.g., biosensor data, strain sensor data such as EMG data or fabric stretch sensor data, image data of user(s) performing different movement(s), user parameter data (e.g., weight, gender), motion capture sensor data, etc.) associated with user(s) who have experienced a musculoskeletal injury to a portion of his or her body (e.g., shoulder, knee, arm, back, neck). Because supervised training is used, the training data is labeled.

Once training is complete, the model is deployed for use as an executable construct that processes an input and provides an output based on the network of nodes and connections defined in the model. The model(s) are stored at one or more databases (e.g., the database 240 of FIG. 2). The model may then be executed by the performance analyzer 224 of the example ergonomic analysis controller 110 of FIG. 2.

Once trained, the deployed model may be operated in an inference phase to process data. In the inference phase, data to be analyzed (e.g., live data) is input to the model, and the model executes to create an output. This inference phase can be thought of as the AI "thinking" to generate the output based on what it learned from the training (e.g., by executing the model to apply the learned patterns and/or associations to the live data). In some examples, input data undergoes pre-processing before being used as an input to the machine learning model. Moreover, in some examples, the output data may undergo post-processing after it is generated by the AI model to transform the output into a useful result (e.g., a display of data, an instruction to be executed by a machine, etc.).

In some examples, output of the deployed model may be captured and provided as feedback. By analyzing the feedback, an accuracy of the deployed model can be determined. If the feedback indicates that the accuracy of the deployed model is less than a threshold or other criterion, training of an updated model can be triggered using the feedback and an updated training data set, hyperparameters, etc., to generate an updated, deployed model.

Referring to FIG. 2, the example system 100 includes a first computing system 226 to train a neural network to predict a likelihood that a user (e.g., the user 102) is experiencing or is likely to experience a strain event with respect to one or more portions of the user's body. The example first computing system 226 includes a neural network processor 228. In examples disclosed herein, the neural network processor 228 implements a neural network.

The example first computing system 226 of FIG. 2 includes a neural network trainer 230. The example neural network trainer 230 of FIG. 2 performs training of the neural network implemented by the neural network processor 228.

The example first computing system 226 of FIG. 2 includes a training controller 232. The example training controller 232 instructs the neural network trainer 230 to perform training of the neural network based on training data 234. In the example of FIG. 2, the training data 234 used by the neural network trainer 230 to train the neural network is stored in a database 236.

In the example of FIG. 2, the training data 234 includes, for example, sensor data (e.g., biosensor data, strain sensor data such as EMG data or fabric stretch sensor data, image data of user(s) performing different movement(s), etc.) associated with user(s) who have experienced a musculoskeletal injury to a portion of his or her body (e.g., shoulder, knee, arm, back, neck). The training data 234 can be specific for particular parts of the body (e.g., EMG data and image data for shoulder movements). The neural network trainer 230 trains the neural network implemented by the neural network processor 228 using the training data 234 to recognize strain event(s) based on the sensor data. In some examples, the training data 234 includes the biosensor data 201, the strain sensor data 202, the environmental data 204, the user profile(s) 212, and/or the population profile(s) 218.

One or more strain event exposure models 238 are generated as a result of the neural network training. For example, a first strain event exposure model 238 can be generated to predict shoulder strain events based on training data associated with shoulder injuries. A second strain event exposure model 238 can be generated to predict knee strain events based on training data associated with knee injuries. The strain event exposure model(s) 238 are stored in a database 240. The databases 236, 240 may be the same storage device or different storage devices.

The performance analyzer 224 of FIG. 2 executes the strain event exposure model(s) 238 for the one or more of the sensor data 201, 202, 204 associated with the user 102 to predict a likelihood that the user 102 is experiencing or is likely to experience a strain event. As disclosed herein, the strain event exposure model(s) 238 can be specific to a portion of the body of interest, such as a shoulder risk exposure model that can be used to identify a risk of strain to a shoulder of the user 102. The strain event exposure model(s) 238 executed by the performance analyzer 224 can be selected based on, for example, a role of the user 102 in the environment 103 (e.g., a shoulder risk exposure model for a user who installs overhead components of an aircraft). Based on the neural network analysis, the performance analyzer 224 generates predicted strain event(s) 242, or predictions of a likelihood that the user 102 is experiencing or is likely to experience a strain event based on the sensor data for which the neural network analysis was performed. The predicted strain event(s) 242 are stored in the database 200.

In some examples, the performance analyzer 224 predicts that the user 102 is at risk for strain event(s) based on the predicted strain event(s) 242 (e.g., based (only) on a prediction generated using real-time sensor data). In other examples, the performance analyzer 224 determines or verifies that the user 102 is at a risk of strain event(s) by comparing the result(s) of the neural network analysis to the user profile(s) 212, previously predicted strain event(s) 242, and/or the population profile(s) 218. For example, execution of the strain event exposure model(s) 238 based on sensor data 201, 202, 204 collected during a first time period may indicate that the user 102 is not experiencing a strain event. However, the performance analyzer 224 may determine that the user 102 is at risk for a strain event based on a comparison of the data 201, 202, 204 collected during the first time period and historical data for the user 102 captured in the user profile(s) 212 indicating changes (e.g., reduction) in user muscle strength over time. Additionally or alternatively, the performance analyzer 224 can determine that the user is experiencing or is likely to experience a strain event based on previously predicted strain event(s) 242, which can indicate that the user 102 is performing a repetitive motion. Thus, the performance analyzer 224 can detect changes indicative of a risk of injury over time based on the neural network analysis and historical data.

In the example of FIG. 2, the strain event exposure model(s) 238 are refined based on data collected from or associated with the user 102 and/or other users (e.g., the population profile(s) 218) over time. Thus, the strain event exposure model(s) 238 are refined based on changes to user-specific data (e.g., changes due to injury of the user 102) and/or trends in the population (e.g., increased occurrence of neck problems from cell phone usage indicating that a neck risk exposure model should be updated to increase predictions of neck strain event(s)). In some examples, the predicted strain event(s) 242 are used to refine the strain event exposure model(s) 238 as part of feedback training

In the example of FIG. 2, if the performance analyzer 224 predicts that the user 102 is experiencing or is likely to experience a strain event with respect to one or more portions of the body of the user 102, the performance analyzer 224 generates instructions for alert(s) to be output to via the output device(s) 112. The alert(s) can include, for instance, haptic feedback alerts, auditory alert(s), text message(s), etc. In some examples, the haptic and/or tactile feedback is presented via the wearable fabric sensor(s) 107 (e.g., via a shirt including haptic feedback sensor(s)) and/or user device 105 associated with the user 102. In some examples, the alert(s) are transmitted to output device(s) 112 associated with third parties (e.g., healthcare provider(s) associated with user 102) based on user setting(s).

In some examples, the ergonomic analysis controller 110 generates recommendations for improving ergonomic form(s). The example ergonomic analysis controller 110 of FIG. 2 includes an ergonomic form recommendation generator 244. In the example of FIG. 2, the ergonomic form recommendation generator 244 generates ergonomic form measure(s) 246 with respect to movement of one or more portions of the body of the user 102 based on user threshold rule(s) 248 and one or more of the sensor data 201, 202, 204, the user profile(s) 212, the population profile(s) 218, and/or the predicted strain event(s) 242. The user threshold rule(s) 248 can define threshold(s) and/or recommendations with respect to user movement based on user characteristics such as gender and age; muscle activity data; and/or biosensor data such as average heartrate, blood pressure, etc. For example, the user threshold rule(s) 248 can define a weight limit (e.g., pounds, kilograms) that is recommended to lifted by a user based on an age of the user and/or sensor data such as average heartrate, blood pressure, muscle tension, etc. In some examples, the user threshold rule(s) 248 are defined based on population profile(s) 218 gathered from a plurality of users.

In the example of FIG. 2, the ergonomic form recommendation generator 244 refines and/or updates the ergonomic form measure(s) 246 based on the predicted strain event(s) 242 and/or changes with respect the sensor data 201, 202, 204 and/or the user profile(s) 212. For example, if strain sensor data 202 from the strain sensor(s) 106, 107 indicates that the user 102 has reduced a duration or intensity of muscle activity (e.g., due to, for instance, an injury or a condition such as arthritis), the ergonomic form recommendation generator 244 updates the ergonomic form measure(s) 246 with respect to, for example, a recommended weight for the user 102 to carry based on the user threshold rule(s) 248. Similarly, the ergonomic form recommendation generator 244 updates the ergonomic measure(s) 246 in response to predictions of a likelihood of strain event(s) by the performance analyzer 224. For instance, the user threshold rule(s) 248 can define that a user should stop performing an activity or reduce a number of repetitions performed in response to the prediction of strain event(s). Thus, the ergonomic form recommendation generator 244 customizes the ergonomic form measure(s) 246 for the user 102 based on data collected from the user 102 over time.

In other examples, the ergonomic form measure(s) 246 include reminders to the user 102 to, for example, check his or her posture when performing a movement. In such examples, the mitigation instruction(s) include audio, visual, and/or haptic feedback reminders to cause the user 102 to be aware of his or her body position, a number of times the movement has been performed, etc. Thus, in some examples, the mitigation measure(s) 246 are generated independent of the sensor data 201, 202, 204.

The ergonomic form recommendation generator 244 transmits the ergonomic form measure(s) 246 for output by the output device(s) 105, 112. The ergonomic form measure(s) 246 can be presented via audio output(s) (e.g., audio output(s) that include a recommended number of repetitions to perform of a movement) and/or visual output(s) (e.g., a visual content in the form of text and/or graphics with respect to a recommended number of repetitions to perform of a movement, an image of a person performing the movement with correct posture, etc.). The ergonomic form recommendation generator 244 can output the ergonomic form measure(s) 246 in response to or independent of the alert(s) generated in response to the prediction of the strain event(s) by the performance analyzer 224.

In some examples, the communicator 214 of the ergonomic analysis controller 110 transmits one or more of the sensor data 201, 202, 204; the user profile(s) 212; and/or the predicted strain event(s) 242 to the output device(s) 112. Also, in some examples, the population data aggregator 114 transmits the population profile(s) 218 to the output device(s) 112. The data can be displayed via user interface(s) accessible by the user 102 and/or by authorized third parties. In some examples, the user interface(s) can display changes over time in the data and/or risk exposure associated with the user 102, compare the user relative to a larger population (e.g., based on the population profile(s) 218), etc.

While an example manner of implementing the ergonomic analysis controller 110 of FIG. 1 is illustrated in FIG. 2, one or more of the elements, processes, and/or devices illustrated in FIG. 2 may be combined, divided, re-arranged, omitted, eliminated and/or implemented in any other way. Further, the example database 200, the example signal modifier 206, the example user profile generator 208, the example communicator 214, the example performance analyzer 224, the example ergonomic form recommendation generator 244, and/or, more generally, the example ergonomic analysis controller 110 of FIG. 2 may be implemented by hardware, software, firmware and/or any combination of hardware, software and/or firmware. Thus, for example, any of the example database 200, the example signal modifier 206, the example user profile generator 208, the example communicator 214, the example performance analyzer 224, the example ergonomic form recommendation generator 244, and/or, more generally, the example ergonomic analysis controller 110 of FIG. 2 could be implemented by one or more analog or digital circuit(s), logic circuits, programmable processor(s), programmable controller(s), graphics processing unit(s) (GPU(s)), digital signal processor(s) (DSP(s)), application specific integrated circuit(s) (ASIC(s)), programmable logic device(s) (PLD(s)) and/or field programmable logic device(s) (FPLD(s)). When reading any of the apparatus or system claims of this patent to cover a purely software and/or firmware implementation, at least one of the example database 200, the example signal modifier 206, the example user profile generator 208, the example communicator 214, the example performance analyzer 224, and/or the example ergonomic form recommendation generator 244 is/are hereby expressly defined to include a non-transitory computer readable storage device or storage disk such as a memory, a digital versatile disk (DVD), a compact disk (CD), a Blu-ray disk, etc. including the software and/or firmware. Further still, the example ergonomic analysis controller 110 may include one or more elements, processes, and/or devices in addition to, or instead of, those illustrated in FIG. 2, and/or may include more than one of any or all of the illustrated elements, processes, and devices. As used herein, the phrase "in communication," including variations thereof, encompasses direct communication and/or indirect communication through one or more intermediary components, and does not require direct physical (e.g., wired) communication and/or constant communication, but rather additionally includes selective communication at periodic intervals, scheduled intervals, aperiodic intervals, and/or one-time events.

While an example manner of implementing the population data aggregator 114 of FIG. 1 is illustrated in FIG. 2, one or more of the elements, processes, and/or devices illustrated in FIG. 2 may be combined, divided, re-arranged, omitted, eliminated and/or implemented in any other way. Further, the example population data aggregator 114 and the example database 220 of FIG. 2 may be implemented by hardware, software, firmware and/or any combination of hardware, software and/or firmware. Thus, for example, the example population data aggregator 114 and the example database 220 of FIG. 2 could be implemented by one or more analog or digital circuit(s), logic circuits, programmable processor(s), programmable controller(s), graphics processing unit(s) (GPU(s)), digital signal processor(s) (DSP(s)), application specific integrated circuit(s) (ASIC(s)), programmable logic device(s) (PLD(s)) and/or field programmable logic device(s) (FPLD(s)). When reading any of the apparatus or system claims of this patent to cover a purely software and/or firmware implementation, at least one of the example population data aggregator 114 and/or the example database 220 is/are hereby expressly defined to include a non-transitory computer readable storage device or storage disk such as a memory, a digital versatile disk (DVD), a compact disk (CD), a Blu-ray disk, etc. including the software and/or firmware. Further still, the example population data aggregator 114 may include one or more elements, processes, and/or devices in addition to, or instead of, those illustrated in FIG. 2, and/or may include more than one of any or all of the illustrated elements, processes, and devices. As used herein, the phrase "in communication," including variations thereof, encompasses direct communication and/or indirect communication through one or more intermediary components, and does not require direct physical (e.g., wired) communication and/or constant communication, but rather additionally includes selective communication at periodic intervals, scheduled intervals, aperiodic intervals, and/or one-time events.

While an example manner of implementing the first computing system 226 is illustrated in FIG. 2, one or more of the elements, processes, and/or devices illustrated in FIG. 2 may be combined, divided, re-arranged, omitted, eliminated and/or implemented in any other way. Further, the example neural network processor 228, the example trainer 230, the example training controller 232, the example database(s) 236, 240 and/or, more generally, the example first computing system 226 of FIG. 2 may be implemented by hardware, software, firmware and/or any combination of hardware, software and/or firmware. Thus, for example, any of the example neural network processor 228, the example trainer 230, the example training controller 232, the example database(s) 236, 240 and/or, more generally, the example first computing system 226 of FIG. 2 could be implemented by one or more analog or digital circuit(s), logic circuits, programmable processor(s), programmable controller(s), graphics processing unit(s) (GPU(s)), digital signal processor(s) (DSP(s)), application specific integrated circuit(s) (ASIC(s)), programmable logic device(s) (PLD(s)) and/or field programmable logic device(s) (FPLD(s)). When reading any of the apparatus or system claims of this patent to cover a purely software and/or firmware implementation, at least one of the example neural network processor 228, the example trainer 230, the example training controller 232, and/or the example database(s) 236, 240 is/are hereby expressly defined to include a non-transitory computer readable storage device or storage disk such as a memory, a digital versatile disk (DVD), a compact disk (CD), a Blu-ray disk, etc. including the software and/or firmware. Further still, the example first computing system 226 may include one or more elements, processes, and/or devices in addition to, or instead of, those illustrated in FIG. 2, and/or may include more than one of any or all of the illustrated elements, processes, and devices. As used herein, the phrase "in communication," including variations thereof, encompasses direct communication and/or indirect communication through one or more intermediary components, and does not require direct physical (e.g., wired) communication and/or constant communication, but rather additionally includes selective communication at periodic intervals, scheduled intervals, aperiodic intervals, and/or one-time events.

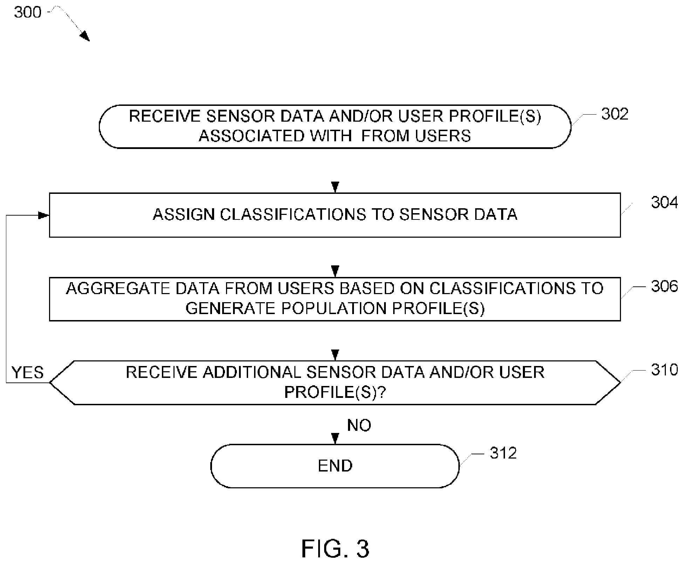

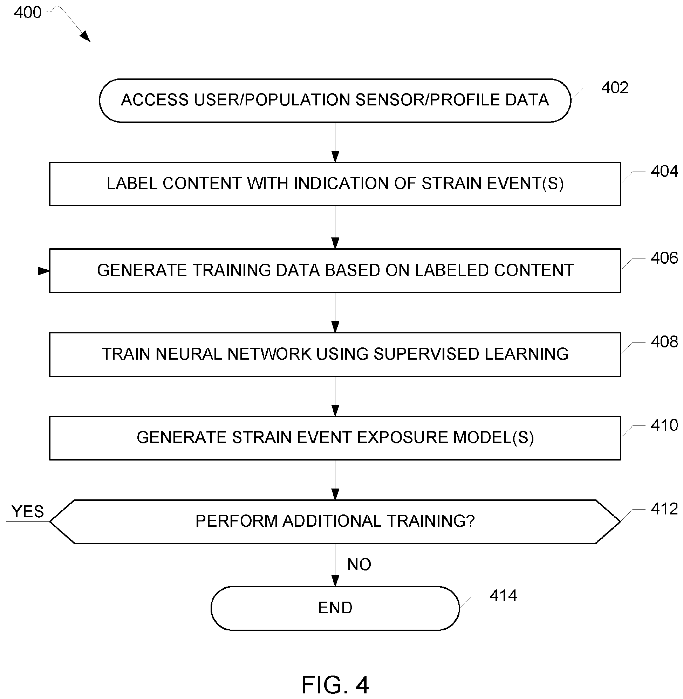

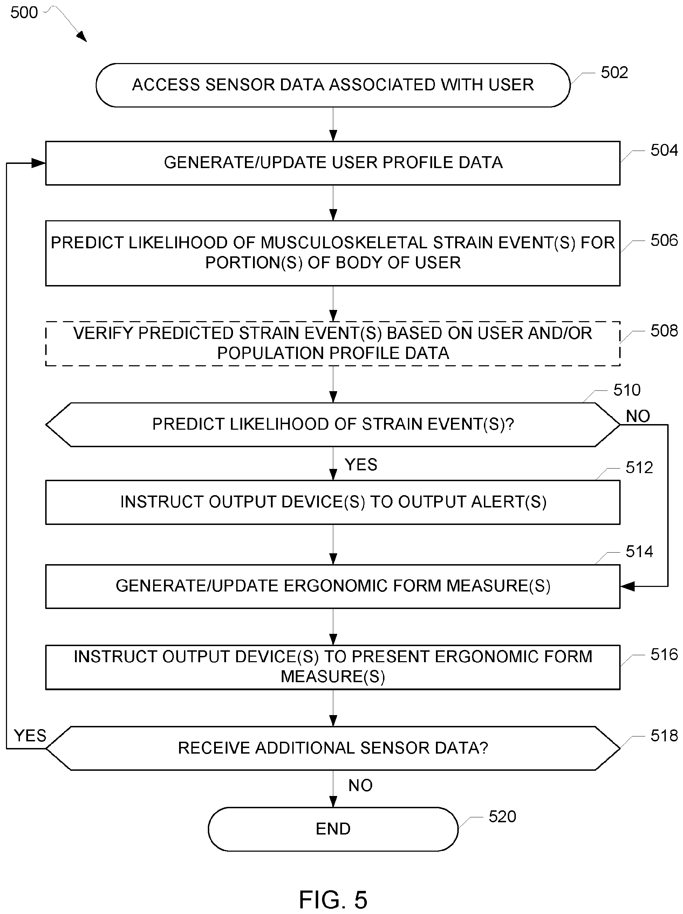

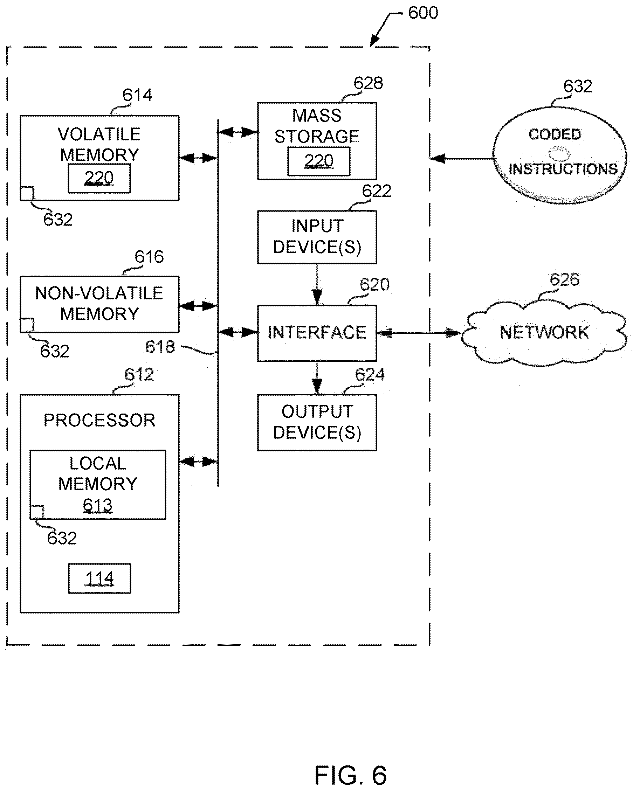

A flowchart representative of example hardware logic, machine readable instructions, hardware implemented state machines, and/or any combination thereof for implementing the example population data aggregator 114 is shown in FIG. 3 A flowchart representative of example hardware logic, machine readable instructions, hardware implemented state machines, and/or any combination thereof for implementing the example first computing system 226 is shown in FIG. 4. A flowchart representative of example hardware logic, machine readable instructions, hardware implemented state machines, and/or any combination thereof for implementing the example ergonomic analysis controller 110 is shown in FIG. 5. The machine readable instructions may be one or more executable programs or portion(s) of an executable program for execution by a computer processor and/or processor circuitry, such as the processor(s) 612, 712, 812 shown in the example processor platform(s) 600, 700, 800 discussed below in connection with FIGS. 6-8. The program(s) may be embodied in software stored on a non-transitory computer readable storage medium such as a CD-ROM, a floppy disk, a hard drive, a DVD, a Blu-ray disk, or a memory associated with the processor(s) 612, 712, 812, but the entire program(s) and/or parts thereof could alternatively be executed by a device other than the processor(s) 612, 712, 812 and/or embodied in firmware or dedicated hardware. Further, although the example program(s) are described with reference to the flowchart(s) illustrated in FIGS. 3-5, many other methods of implementing the example population data aggregator 114, the example first computing system 226, and/or the example ergonomic analysis controller 110 may alternatively be used. For example, the order of execution of the blocks may be changed, and/or some of the blocks described may be changed, eliminated, or combined. Additionally or alternatively, any or all of the blocks may be implemented by one or more hardware circuits (e.g., discrete and/or integrated analog and/or digital circuitry, an FPGA, an ASIC, a comparator, an operational-amplifier (op-amp), a logic circuit, etc.) structured to perform the corresponding operation without executing software or firmware. The processor circuitry may be distributed in different network locations and/or local to one or more devices (e.g., a multi-core processor in a single machine, multiple processors distributed across a server rack, etc.).

The machine readable instructions described herein may be stored in one or more of a compressed format, an encrypted format, a fragmented format, a compiled format, an executable format, a packaged format, etc. Machine readable instructions as described herein may be stored as data or a data structure (e.g., portions of instructions, code, representations of code, etc.) that may be utilized to create, manufacture, and/or produce machine executable instructions. For example, the machine readable instructions may be fragmented and stored on one or more storage devices and/or computing devices (e.g., servers) located at the same or different locations of a network or collection of networks (e.g., in the cloud, in edge devices, etc.). The machine readable instructions may require one or more of installation, modification, adaptation, updating, combining, supplementing, configuring, decryption, decompression, unpacking, distribution, reassignment, compilation, etc. in order to make them directly readable, interpretable, and/or executable by a computing device and/or other machine. For example, the machine readable instructions may be stored in multiple parts, which are individually compressed, encrypted, and stored on separate computing devices, wherein the parts when decrypted, decompressed, and combined form a set of executable instructions that implement one or more functions that may together form a program such as that described herein.

In other examples, the machine readable instructions may be stored in a state in which they may be read by processor circuitry, but require addition of a library (e.g., a dynamic link library (DLL)), a software development kit (SDK), an application programming interface (API), etc. in order to execute the instructions on a particular computing device or other device. In other examples, the machine readable instructions may need to be configured (e.g., settings stored, data input, network addresses recorded, etc.) before the machine readable instructions and/or the corresponding program(s) can be executed in whole or in part. Thus, machine readable media, as used herein, may include machine readable instructions and/or program(s) regardless of the particular format or state of the machine readable instructions and/or program(s) when stored or otherwise at rest or in transit.

The machine readable instructions described herein can be represented by any past, present, or future instruction language, scripting language, programming language, etc. For example, the machine readable instructions may be represented using any of the following languages: C, C++, Java, C#, Perl, Python, JavaScript, HyperText Markup Language (HTML), Structured Query Language (SQL), Swift, etc.

As mentioned above, the example processes of FIGS. 3-5 may be implemented using executable instructions (e.g., computer and/or machine readable instructions) stored on a non-transitory computer and/or machine readable medium such as a hard disk drive, a flash memory, a read-only memory, a compact disk, a digital versatile disk, a cache, a random-access memory and/or any other storage device or storage disk in which information is stored for any duration (e.g., for extended time periods, permanently, for brief instances, for temporarily buffering, and/or for caching of the information). As used herein, the term non-transitory computer readable medium is expressly defined to include any type of computer readable storage device and/or storage disk and to exclude propagating signals and to exclude transmission media.

"Including" and "comprising" (and all forms and tenses thereof) are used herein to be open ended terms. Thus, whenever a claim employs any form of "include" or "comprise" (e.g., comprises, includes, comprising, including, having, etc.) as a preamble or within a claim recitation of any kind, it is to be understood that additional elements, terms, etc. may be present without falling outside the scope of the corresponding claim or recitation. As used herein, when the phrase "at least" is used as the transition term in, for example, a preamble of a claim, it is open-ended in the same manner as the term "comprising" and "including" are open ended. The term "and/or" when used, for example, in a form such as A, B, and/or C refers to any combination or subset of A, B, C such as (1) A alone, (2) B alone, (3) C alone, (4) A with B, (5) A with C, (6) B with C, and (7) A with B and with C. As used herein in the context of describing structures, components, items, objects and/or things, the phrase "at least one of A and B" is intended to refer to examples including any of (1) at least one A, (2) at least one B, and (3) at least one A and at least one B. Similarly, as used herein in the context of describing structures, components, items, objects and/or things, the phrase "at least one of A or B" is intended to refer to examples including any of (1) at least one A, (2) at least one B, and (3) at least one A and at least one B. As used herein in the context of describing the performance or execution of processes, instructions, actions, activities and/or steps, the phrase "at least one of A and B" is intended to refer to examples including any of (1) at least one A, (2) at least one B, and (3) at least one A and at least one B. Similarly, as used herein in the context of describing the performance or execution of processes, instructions, actions, activities and/or steps, the phrase "at least one of A or B" is intended to refer to examples including any of (1) at least one A, (2) at least one B, and (3) at least one A and at least one B.

As used herein, singular references (e.g., "a", "an", "first", "second", etc.) do not exclude a plurality. The term "a" or "an" entity, as used herein, refers to one or more of that entity. The terms "a" (or "an"), "one or more", and "at least one" can be used interchangeably herein. Furthermore, although individually listed, a plurality of means, elements or method actions may be implemented by, e.g., a single unit or processor. Additionally, although individual features may be included in different examples or claims, these may possibly be combined, and the inclusion in different examples or claims does not imply that a combination of features is not feasible and/or advantageous.

FIG. 3 is a flowchart representative of example machine readable instructions 300 that, when executed by the population data aggregator 114 of the example system 100 of FIGS. 1 and/or 2, cause the population data aggregator 114 to generate population profile(s) 218 based on sensor data associated with a plurality of user(s). The example instructions 300 begin with the population data aggregator 114 receiving sensor data and/or respective user profiles associated a plurality of users (block 302). For example, the population data aggregator 114 can receive biosensor data from one or more users (e.g., the biosensor data 201), strain sensor data from one or more users (e.g., the strain sensor data 202), and/or environmental data (e.g., the environmental data 204) indicative of conditions in an environment and/or associated with user(s) in the environment. In some examples, the population data aggregator 114 receives user profile(s) indicative of historical or reference data for the user(s).

The population data aggregator 114 assigns classifications to the data (e.g., the sensor data 201, 202, 204, the user profile(s) 212) based on the population data classification rule(s) 222 (block 304). For example, the population data aggregator 114 can classify biosensor data received from a user based on data type (e.g., heart rate data) and user properties (e.g., age, gender). As another example, the population data aggregator 114 can classify environmental data based on data type (e.g., image data) and environment type (e.g., factory, office, etc.).

The population data aggregator 114 aggregates data from two or more users based on the classifications to generate the population profile(s) 218 (block 306). The population profile(s) 218 are stored in the database 220 associated with the population data aggregator 114.

If additional sensor data and/or user profile data is received from user(s), the population data aggregator 114 continues to classify and aggregate the data to generate and/or update the population profile(s) 218 (block 308). The instructions 300 of FIG. 3 end when no further sensor data and/or user profile data is received (block 310).

FIG. 4 is a flowchart representative of example machine readable instructions 400 that, when executed by the example first computing system 226 of FIG. 2, cause the first computing system 226 to train a neural network to predict a likelihood of musculoskeletal strain event(s) with respect to one or more portions of a body of a user. The example instructions 400 of FIG. 4, when executed by the first computing system 226 of FIG. 2, result in a neural network and/or a model thereof, that can be distributed to other computing systems, such as the performance analyzer 224 of the example ergonomic analysis controller 110 of FIG. 2.

The example instructions 400 begin with the training controller 232 accessing sensor data and/or profile data associated with user(s) and/or population(s) stored in the database 236 (block 402). The sensor data can include, for example, one or more of previously generated biosensor data 201, strain sensor data 202, environmental data 204, user profile(s) 212, and/or population profile(s) 218. In some examples, the data includes the previously predicted strain event(s) 242 generated by the performance analyzer 224 as part of feedback training. In some examples, the sensor data is associated with a particular portion of the body of interest with respect to strain events, such as a shoulder, a knee, a wrist, neck, back, etc.

The example training controller 232 labels the data as indicative of strain event(s) (block 404). For example, when the sensor data includes image data of a user performing a movement, the training controller 232 labels the image(s) corresponding to the user in a position in which one or more portions of the user's body is stressed and/or strained such that an injury could occur. In other examples, the training controller 232 labels muscle tension data with thresholds for detecting strain events based on, for example, previously generated or known reference data including, for instance, anthropometric data, population data generated by the population data aggregator 114, etc.

The example training controller 232 generates the training data 234 based on the labeled sensor data (block 406).

The example training controller 232 instructs the neural network trainer 230 to perform training of the neural network 228 using the training data 234 (block 408). In the example of FIG. 4, the training is based on supervised learning. As a result of the training, the strain event exposure model(s) 238 are generated (block 410). Based on the strain event exposure model(s) 238, the neural network is trained to predict a likelihood that a user is experiencing or is likely to experience a strain event at one or more portions of the user's body (e.g., shoulder, knee, arm, back, neck). The strain event exposure model(s) 238 can be stored in the database 240 for access by the performance analyzer 224 of the ergonomic analysis controller 110 of FIG. 2. The example instructions 400 of FIG. 4 end when no additional training (e.g., retraining) is to be performed (blocks 412, 414).

FIG. 5 is a flowchart representative of example machine readable instructions 500 that, when executed by the ergonomic analysis controller 110 of FIGS. 1 and/or 2, cause the ergonomic analysis controller 110 to predict a likelihood of musculoskeletal strain event(s) with respect to one or more portions of a body of a user (e.g., the user 102). The example instructions 500 can be executed by one or more processor(s) of user device(s) and/or cloud-based device(s).

The example instructions 500 begin with the ergonomic analysis controller 110 accessing sensor data associated with a user (e.g., the user 102 of FIG. 1). The sensor data can include biosensor data 201, strain sensor data 202, and/or environmental data 204 (e.g., data collected with respect to the environment 103 and/or the user in the environment 103, such as images of the user in the environment) (block 502). The user profile generator 208 of the ergonomic analysis controller 110 of FIG. 2 generates and/or updates user profile(s) 212 based on the sensor data 201, 202, 204 stored in the database 200 (block 504). The user profile(s) 212 can serve as baseline or reference data for the user.

The performance analyzer 224 predicts a likelihood that is the user is experiencing or is likely to experience a strain event with respect to one or more portions of the body of the user (block 506). In some examples, the performance analyzer 224 executes the strain event exposure model(s) 238 based on one or more of the sensor data 201, 202, 204 to predict a likelihood that is the user is experiencing or is likely to experience a strain event with respect to one or more portions of the body of the user. In some examples, the performance analyzer 224 verifies the predicted strain event(s) 242 in view of the user profile(s) 212 and/or the population profile(s) 218 (block 508). For example, the performance analyzer 224 can confirm a likelihood that the user is experiencing strain event or is likely to experience a strain event by comparing the sensor data 201, 202, 204 used in the neural network analysis to the historical or baseline user profile data 212. In some examples, the performance analyzer 224 verifies the prediction of the strain event(s) by comparing the sensor data 201, 202, 204 to the population profile(s) 218 generated by population data aggregator 114.

Additionally or alternatively, at block 506, the performance analyzer 224 can predict a likelihood that is the user is experiencing or is likely to experience a strain event with respect to one or more portions of the body of the user by mapping parameter(s) of the user such as weight, age, gender, etc. to the population profile(s) 218 to determine, for instance, an average or optimal amount of weight to be lifted by the user based on average data for other users having similar profiles; an optimal range of motion of a shoulder of the user based on other users having similar medical conditions such as arthritis, etc. The performance analyzer 224 can compare the average or optimal ergonomic data from the population profile(s) with the sensor data 201, 202, 204 to determine if the user is experiencing or is likely to experience a strain event.

If the performance analyzer 224 predicts a likelihood of strain event(s) for one or more portions of the user's body (block 510), the performance analyzer 224 instructs the output device(s) 112 (e.g., a smartphone) to output alert(s) to alert the user as to the strain event(s) (block 512). The alert(s) can include audio, visual, and/or haptic feedback alert(s).

In the example of FIG. 5, the ergonomic form recommendation generator 244 generates (or updates) ergonomic form measure(s) 246 for the user based on the sensor data 201, 202, 204 and/or in response to the prediction of the strain event(s) (block 514). Also, in the example of FIG. 5, if the performance analyzer 224 does not determine a likelihood of a strain event (block 510), the ergonomic analysis controller 110 generates ergonomic form measure(s) 246 to guide the user with respect to movement(s) and/or positions that promote and/or preserve musculoskeletal integrity. The ergonomic form recommendation generator 244 generates the ergonomic form measure(s) 246 based on the user threshold rule(s) 248 and the sensor data 201, 202, 204, the user profile(s) 212 and/or the population profile(s) 218. The ergonomic form measure(s) 246 can define, for example, recommended number of repetitions of an activity to be performed to reduce a likelihood of a strain event.

The ergonomic form recommendation generator 244 instructs the output device(s) 112 to output the ergonomic form measure(s) 246 for presentation to the user (block 516). The ergonomic form measure(s) 246 can be presented in visual and/or audio format, for example.

The ergonomic analysis controller 110 continues to update the user profile data 212, predict a likelihood of strain event(s), and provide ergonomic form measure(s) as additional sensor data is received by the ergonomic analysis controller 110 (block 518). The example instructions 500 of FIG. 5 end when no further sensor data 201, 202, 204 is received (block 520).

FIG. 6 is a block diagram of an example processor platform 600 structured to execute the instructions of FIG. 3 to implement the example population data aggregator 114 of FIGS. 1 and/or 2. The processor platform 600 can be, for example, a server, a personal computer, a workstation, a self-learning machine (e.g., a neural network), a mobile device (e.g., a cell phone, a smart phone, a tablet such as an iPadTM), a personal digital assistant (PDA), an Internet appliance, a headset or other wearable device, or any other type of computing device.

The processor platform 600 of some examples including the illustrated example includes a processor 612. The processor 612 of some examples including the illustrated example is hardware. For example, the processor 612 can be implemented by one or more integrated circuits, logic circuits, microprocessors, GPUs, DSPs, or controllers from any desired family or manufacturer. The hardware processor may be a semiconductor based (e.g., silicon based) device. In this and other examples, the processor implements the example population data aggregator 114.

The processor 612 of some examples including the illustrated example includes a local memory 613 (e.g., a cache). The processor 612 of some examples including the illustrated example is in communication with a main memory including a volatile memory 614 and a non-volatile memory 616 via a bus 618. The volatile memory 614 may be implemented by Synchronous Dynamic Random Access Memory (SDRAM), Dynamic Random Access Memory (DRAM), RAMBUS^{®} Dynamic Random Access Memory (RDRAM^{®}) and/or any other type of random access memory device. The non-volatile memory 616 may be implemented by flash memory and/or any other desired type of memory device. Access to the main memory 614, 616 is controlled by a memory controller.

The processor platform 600 of some examples including the illustrated example also includes an interface circuit 620. The interface circuit 620 may be implemented by any type of interface standard, such as an Ethernet interface, a universal serial bus (USB), a Bluetooth^{®} interface, a near field communication (NFC) interface, and/or a PCI express interface.

In some examples including the illustrated example, one or more input devices 622 are connected to the interface circuit 620. The input device(s) 622 permit(s) a user to enter data and/or commands into the processor 612. The input device(s) can be implemented by, for example, an audio sensor, a microphone, a camera (still or video), a keyboard, a button, a mouse, a touchscreen, a track-pad, a trackball, isopoint and/or a voice recognition system.

One or more output devices 624 are also connected to the interface circuit 620 of some examples including the illustrated example. The output devices 624 can be implemented, for example, by display devices (e.g., a light emitting diode (LED), an organic light emitting diode (OLED), a liquid crystal display (LCD), a cathode ray tube display (CRT), an in-place switching (IPS) display, a touchscreen, etc.), a tactile output device, a printer and/or speaker. The interface circuit 620 of some examples including the illustrated example, thus, typically includes a graphics driver card, a graphics driver chip and/or a graphics driver processor.

The interface circuit 620 of some examples including the illustrated example also includes a communication device such as a transmitter, a receiver, a transceiver, a modem, a residential gateway, a wireless access point, and/or a network interface to facilitate exchange of data with external machines (e.g., computing devices of any kind) via a network 626. The communication can be via, for example, an Ethernet connection, a digital subscriber line (DSL) connection, a telephone line connection, a coaxial cable system, a satellite system, a line-of-site wireless system, a cellular telephone system, etc.

The processor platform 600 of some examples including the illustrated example also includes one or more mass storage devices 628 for storing software and/or data. Examples of such mass storage devices 628 include floppy disk drives, hard drive disks, compact disk drives, Blu-ray disk drives, redundant array of independent disks (RAID) systems, and digital versatile disk (DVD) drives.

Coded instructions 632 of FIG. 6 may be stored in the mass storage device 628, in the volatile memory 614, in the non-volatile memory 616, and/or on a removable non-transitory computer readable storage medium such as a CD or DVD.

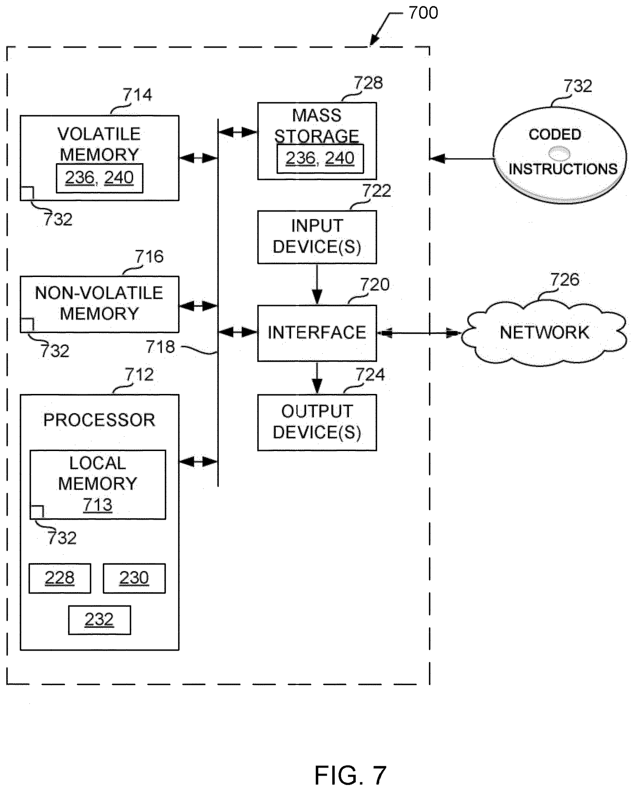

FIG. 7 is a block diagram of an example processor platform 700 structured to execute the instructions of FIG. 4 to implement the first computing system 226 of FIG. 2. The processor platform 700 can be, for example, a server, a personal computer, a workstation, a self-learning machine (e.g., a neural network), an Internet appliance, or any other type of computing device.

The processor platform 700 of some examples including the illustrated example includes a processor 712. The processor 712 of some examples including the illustrated example is hardware. For example, the processor 712 can be implemented by one or more integrated circuits, logic circuits, microprocessors, GPUs, DSPs, or controllers from any desired family or manufacturer. The hardware processor may be a semiconductor based (e.g., silicon based) device. In this and other examples, the processor implements the example neural network processor 228, the example trainer 230, and the example training controller 232.

The processor 712 of some examples including the illustrated example includes a local memory 713 (e.g., a cache). The processor 712 of some examples including the illustrated example is in communication with a main memory including a volatile memory 714 and a non-volatile memory 716 via a bus 718. The volatile memory 714 may be implemented by Synchronous Dynamic Random Access Memory (SDRAM), Dynamic Random Access Memory (DRAM), RAMBUS^{®} Dynamic Random Access Memory (RDRAM^{®}) and/or any other type of random access memory device. The non-volatile memory 716 may be implemented by flash memory and/or any other desired type of memory device. Access to the main memory 714, 716 is controlled by a memory controller.

The processor platform 700 of some examples including the illustrated example also includes an interface circuit 720. The interface circuit 720 may be implemented by any type of interface standard, such as an Ethernet interface, a universal serial bus (USB), a Bluetooth^{®} interface, a near field communication (NFC) interface, and/or a PCI express interface.

In some examples including the illustrated example, one or more input devices 722 are connected to the interface circuit 720. The input device(s) 722 permit(s) a user to enter data and/or commands into the processor 712. The input device(s) can be implemented by, for example, an audio sensor, a microphone, a camera (still or video), a keyboard, a button, a mouse, a touchscreen, a track-pad, a trackball, isopoint and/or a voice recognition system.

One or more output devices 724 are also connected to the interface circuit 720 of some examples including the illustrated example. The output devices 724 can be implemented, for example, by display devices (e.g., a light emitting diode (LED), an organic light emitting diode (OLED), a liquid crystal display (LCD), a cathode ray tube display (CRT), an in-place switching (IPS) display, a touchscreen, etc.), a tactile output device, a printer and/or speaker. The interface circuit 720 of some examples including the illustrated example, thus, typically includes a graphics driver card, a graphics driver chip and/or a graphics driver processor.

The interface circuit 720 of some examples including the illustrated example also includes a communication device such as a transmitter, a receiver, a transceiver, a modem, a residential gateway, a wireless access point, and/or a network interface to facilitate exchange of data with external machines (e.g., computing devices of any kind) via a network 726. The communication can be via, for example, an Ethernet connection, a digital subscriber line (DSL) connection, a telephone line connection, a coaxial cable system, a satellite system, a line-of-site wireless system, a cellular telephone system, etc.

The processor platform 700 of some examples including the illustrated example also includes one or more mass storage devices 728 for storing software and/or data. Examples of such mass storage devices 728 include floppy disk drives, hard drive disks, compact disk drives, Blu-ray disk drives, redundant array of independent disks (RAID) systems, and digital versatile disk (DVD) drives.

Coded instructions 732 of FIG. 7 may be stored in the mass storage device 728, in the volatile memory 714, in the non-volatile memory 716, and/or on a removable non-transitory computer readable storage medium such as a CD or DVD.

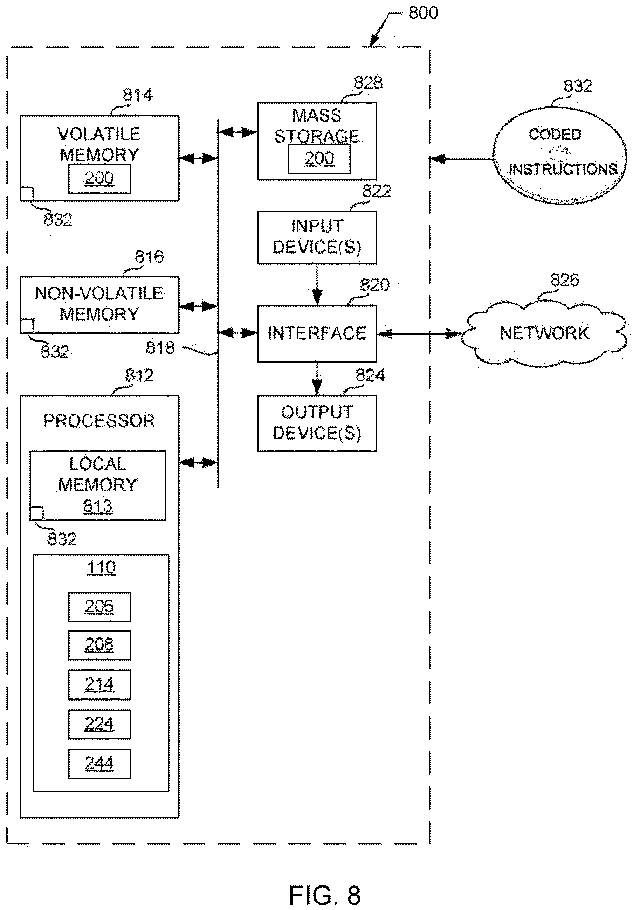

FIG. 8 is a block diagram of an example processor platform 800 structured to execute the instructions of FIG. 5 to implement the example ergonomic analysis controller 110 of FIGS. 1 and/or 2. The processor platform 800 can be, for example, a server, a personal computer, a workstation, a self-learning machine (e.g., a neural network), a mobile device (e.g., a cell phone, a smart phone, a tablet such as an iPad^{™}), a personal digital assistant (PDA), an Internet appliance, a headset or other wearable device, or any other type of computing device.

The processor platform 800 of some examples including the illustrated example includes a processor 812. The processor 812 of some examples including the illustrated example is hardware. For example, the processor 812 can be implemented by one or more integrated circuits, logic circuits, microprocessors, GPUs, DSPs, or controllers from any desired family or manufacturer. The hardware processor may be a semiconductor based (e.g., silicon based) device. In this and other examples, the processor implements the example signal modifier 206, example user profile generator 208, the example communicator 214, the example performance analyzer 224, and the example ergonomic form recommendation generator 244.

The processor 812 of some examples including the illustrated example includes a local memory 813 (e.g., a cache). The processor 812 of some examples including the illustrated example is in communication with a main memory including a volatile memory 814 and a non-volatile memory 816 via a bus 818. The volatile memory 814 may be implemented by Synchronous Dynamic Random Access Memory (SDRAM), Dynamic Random Access Memory (DRAM), RAMBUS^{®} Dynamic Random Access Memory (RDRAM^{®}) and/or any other type of random access memory device. The non-volatile memory 816 may be implemented by flash memory and/or any other desired type of memory device. Access to the main memory 814, 816 is controlled by a memory controller.

The processor platform 800 of some examples including the illustrated example also includes an interface circuit 820. The interface circuit 820 may be implemented by any type of interface standard, such as an Ethernet interface, a universal serial bus (USB), a Bluetooth^{®} interface, a near field communication (NFC) interface, and/or a PCI express interface.

In some examples including the illustrated example, one or more input devices 822 are connected to the interface circuit 820. The input device(s) 822 permit(s) a user to enter data and/or commands into the processor 812. The input device(s) can be implemented by, for example, an audio sensor, a microphone, a camera (still or video), a keyboard, a button, a mouse, a touchscreen, a track-pad, a trackball, isopoint and/or a voice recognition system.

One or more output devices 824 are also connected to the interface circuit 820 of some examples including the illustrated example. The output devices 824 can be implemented, for example, by display devices (e.g., a light emitting diode (LED), an organic light emitting diode (OLED), a liquid crystal display (LCD), a cathode ray tube display (CRT), an in-place switching (IPS) display, a touchscreen, etc.), a tactile output device, a printer and/or speaker. The interface circuit 820 of some examples including the illustrated example, thus, typically includes a graphics driver card, a graphics driver chip and/or a graphics driver processor.

The interface circuit 820 of some examples including the illustrated example also includes a communication device such as a transmitter, a receiver, a transceiver, a modem, a residential gateway, a wireless access point, and/or a network interface to facilitate exchange of data with external machines (e.g., computing devices of any kind) via a network 826. The communication can be via, for example, an Ethernet connection, a digital subscriber line (DSL) connection, a telephone line connection, a coaxial cable system, a satellite system, a line-of-site wireless system, a cellular telephone system, etc.

The processor platform 800 of some examples including the illustrated example also includes one or more mass storage devices 828 for storing software and/or data. Examples of such mass storage devices 828 include floppy disk drives, hard drive disks, compact disk drives, Blu-ray disk drives, redundant array of independent disks (RAID) systems, and digital versatile disk (DVD) drives.
Coded instructions 832 of FIG. 8 may be stored in the mass storage device 828, in the volatile memory 814, in the non-volatile memory 816, and/or on a removable non-transitory computer readable storage medium such as a CD or DVD.

From the foregoing, it will be appreciated that example systems, methods, apparatus, and articles of manufacture have been disclosed that predict a likelihood that a user is experiencing or is likely to experience a musculoskeletal strain event at one or more portions of the user's body (e.g., shoulder, knee, wrist, arm, back, neck) and alert the user in response to the predicted strain event(s). Examples disclosed herein implement neural network model(s) to predict the likelihood of the strain event(s) based on sensor data associated with the user, such as biosensor data, strain sensor data, and/or data from sensor(s) in the environment in which the user is located that capture data associated with the user (e.g., image data of the user) and/or conditions in the environment (e.g., vibrations). Example neural network(s) are developed and/or refined based on data collected from the user and/or other user(s) over time. As such, rather than relying on static reference data, examples disclosed herein dynamically respond to changes in user and/or movement characteristics to provide customized detection of strain event(s) and optimized ergonomic form recommendations for the user.

Although certain example methods, apparatus and articles of manufacture have been disclosed herein, the scope of coverage of this patent is not limited thereto. On the contrary, this patent covers all methods, apparatus and articles of manufacture fairly falling within the scope of the claims of this patent.

## Claims

1. An apparatus comprising:
a user profile generator (208) configured to generate a user profile (212) for a user based on strain sensor data (202) collected via one or more strain sensors (106, 107) associated with the user, the user profile including the strain sensor data, historical sensor data for the user, and user parameter data;
an aggregator (114) configured to aggregate the strain sensor data for the user (102) with strain sensor data for a population of users to generate a population profile (218) comprising population data;
a performance analyzer (224) configured to:
determine a likelihood of the user (102) experiencing a musculoskeletal strain event at a portion of the body of the user (102) based on the strain sensor data (202) and by comparing the user parameter data stored in the user profile (212) to the population profile (218), wherein the performance analyzer (224) predicts that the user is at risk of a musculoskeletal strain event (242) if the strain sensor data (201) indicates that the portion of the body of the user (102) is under a strain which exceeds an amount associated with an average or an optimal exertion by the user based on the comparison of the user parameter data and the population data; and
transmit, in response to the prediction of the musculoskeletal strain event, an instruction including an alert to be output by an output device (112); and
an ergonomic form recommendation generator (244) configured to transmit, in response to the prediction of the musculoskeletal strain event, an instruction including an ergonomic form measure (246) to be output by the output device, wherein the ergonomic form measure is generated based on the user profile.

2. The apparatus of claim 1, wherein the user parameter data comprises one or more characteristics of the user including at least one of: age, gender, race, weight, athletic ability, and medical conditions.

3. The apparatus of claim 1 or 2, wherein the performance analyzer is configured to predict the musculoskeletal strain event based on biosensor data (201) collected via one or more biosensors (104) associated with the user.

4. The apparatus of any of claims 1-3, wherein: the ergonomic form recommendation generator is configured to further generate the ergonomic form measure based on a rule (248) defining a threshold associated with movement by the user.

5. The apparatus of any of claims 1-4, wherein the performance analyzer is configured to execute a neural network model (238) to predict the musculoskeletal strain event.

6. The apparatus of any of claims 1-5, wherein the alert includes one or more of a visual alert, an audio alert, or a haptic feedback alert.

7. The apparatus of any of claims 1-6, wherein at least one of the one or more strain sensors is carried by a wearable fabric (107).

8. The apparatus of any of claims 1-7, including an additional sensor (108), the additional sensor including a camera (108) configured to capture image data (204) of the user in an environment (103), and wherein the performance analyzer is configured:
to update the user profile (212) for the user based on one or more of the strain sensor data or the image data (204).

9. The apparatus of any previous claim, wherein the user is a first user and the ergonomic form controller is configured to transmit the strain sensor data and image data to the aggregator, the aggregator configured to aggregate the strain sensor data and image data with second strain sensor data and second image data associated with a second user to generate the population profile (218).

10. A method comprising:
generating a user profile (212) for a user based on strain sensor data collected via one or more strain sensors (106, 107) associated with the user, the user profile including the strain sensor data, historical sensor data for the user, and user parameter data;
aggregating the strain sensor data for the user with strain sensor data for a population of users to generate a population profile (218) comprising population data;
determining a likelihood of the user (102) experiencing a musculoskeletal strain event at a portion of the body of the user (102) based on the strain sensor data (202) and by comparing the user parameter data stored in the user profile (212) to the population profile (218);
predicting that the user is at risk of a musculoskeletal strain event (242) if the strain sensor data (201) indicates that the portion of the body of the user (102) is under a strain which exceeds an amount associated with an average or an optimal exertion by the user based on the comparison of the user parameter data and the population data;
and
transmitting, in response to the prediction of the musculoskeletal strain event, an instruction including an alert and an ergonomic form measure (246) to be output by an output device (112), wherein the ergonomic form measure is generated based on the user profile.

11. The method of claim 10, wherein the user parameter data comprises one or more characteristics of the user including at least one of: age, gender, race, weight, athletic ability, and any medical conditions.

12. The method of claim 10 or 11, wherein the predicting of the musculoskeletal strain event is based on biosensor data (201) collected via one or more biosensors (104) associated with the user.

13. The method of any of claims 10-12, further including:
generating the ergonomic form measure based on a rule (248) defining a threshold associated with movement by the user.

14. The method of any of claims 10-13, wherein the predicting the musculoskeletal strain event includes executing a neural network model (238).

15. A computer program comprising computer program instructions that, when executed by a computer processor, cause the computer processor to execute the method of any of claims 10-14, or a non-transitory computer-readable medium having stored thereon such a computer program.

## Patentansprüche

1. Einrichtung, umfassend:
einen Benutzerprofilgenerator (208), der so konfiguriert ist, dass er ein Benutzerprofil (212) für einen Benutzer basierend auf Dehnungssensordaten (202) generiert, die über einen oder mehrere dem Benutzer zugeordnete Dehnungssensoren (106, 107) erfasst wurden, wobei das Benutzerprofil die Dehnungssensordaten, historische Sensordaten für den Benutzer und Benutzerparameterdaten einschließt;
einen Aggregator (114), der so konfiguriert ist, dass er die Dehnungssensordaten für den Benutzer (102) mit Dehnungssensordaten für eine Benutzerpopulation aggregiert, um ein Populationsprofil (218) zu erstellen, das Populationsdaten umfasst;
einen Leistungsanalysator (224), der konfiguriert ist, zum:
Bestimmen der Wahrscheinlichkeit, dass der Benutzer (102) an einem Körperteil des Benutzers (102) ein muskoloskelettalen Dehnungsereignis erleidet, anhand der Dehnungssensordaten (202) und durch Vergleich der im Benutzerprofil (212) gespeicherten Benutzerparameterdaten mit dem Populationsprofil (218), wobei der Leistungsanalysator (224) vorhersagt, dass der Benutzer einem Risiko für ein muskoloskelettalen Dehnungsereignis (242) ausgesetzt ist, wenn die Dehnungssensordaten (201) darauf hinweisen, dass der Körperteil des Benutzers (102) einer Dehnung ausgesetzt ist, die einen Wert überschreitet, der einer durchschnittlichen oder optimalen Belastung des Benutzers entspricht, basierend auf dem Vergleich der Benutzerparameterdaten und der Populationsdaten; und
Übertragen, als Reaktion auf die Vorhersage eines muskoloskelettalen Dehnungsereignisses, einer Anweisung, die eine Warnung einschließt, die von einer Ausgabevorrichtung ausgegeben werden soll (112); und
einen ergonomischen Formempfehlungsgenerator (244), der so konfiguriert ist, dass er als Reaktion auf die Vorhersage des muskoloskelettalen Dehnungsereignisses eine Anweisung einschließlich eines ergonomischen Formmaßes (246) überträgt, die von der Ausgabevorrichtung ausgegeben werden soll, wobei das ergonomische Formmaß auf Basis des Benutzerprofils generiert wird.

2. Einrichtung nach Anspruch 1, wobei die Benutzerparameterdaten ein oder mehrere Merkmale des Benutzers umfassen, einschließlich mindestens eines der folgenden: Alter, Geschlecht, Rasse, Gewicht, sportliche Fähigkeiten und medizinische Zustände.

3. Einrichtung nach Anspruch 1 oder 2, wobei der Leistungsanalysator so konfiguriert ist, dass er das muskoloskelettale Dehnungsereignis basierend auf Biosensordaten (201) vorhersagt, die über einen oder mehrere dem Benutzer zugeordnete Biosensoren (104) erfasst werden.

4. Einrichtung nach einem der Ansprüche 1-3, wobei der ergonomische Formempfehlungsgenerator so konfiguriert ist, dass er weiter das ergonomische Formmaß auf Basis einer Regel (248) generiert, die einen mit der Bewegung des Benutzers verbundenen Schwellenwert definiert.

5. Einrichtung nach einem der Ansprüche 1-4, wobei der Leistungsanalysator so konfiguriert ist, dass er ein neuronales Netzwerkmodell (238) ausführt, um das muskoloskelettale Dehnungsereignis vorherzusagen.

6. Einrichtung nach einem der Ansprüche 1-5, wobei die Warnung eines oder mehrere von einer visuellen Warnung, einer akustischen Warnung oder einer haptischen Rückmeldung einschließt.

7. Einrichtung nach einem der Ansprüche 1-6, wobei mindestens einer des einen oder mehreren Dehnungssensoren von einem tragbaren Textilgewebe (107) getragen wird.

8. Einrichtung nach einem der Ansprüche 1-7, einschließlich eines zusätzlichen Sensors (108), wobei der zusätzliche Sensor eine Kamera (108) einschließt, die so konfiguriert ist, dass sie Bilddaten (204) des Benutzers in einer Umgebung (103) erfasst, und wobei der Leistungsanalysator konfiguriert ist, zum:
Aktualisieren des Benutzerprofils (212) für den Benutzer auf Basis eines oder mehrerer Dehnungssensordaten oder der Bilddaten (204).

9. Einrichtung nach einem vorstehenden Anspruch, wobei der Benutzer ein erster Benutzer ist und die ergonomische Formsteuereinheit so konfiguriert ist, dass sie die Dehnungssensordaten und Bilddaten an den Aggregator überträgt, wobei der Aggregator so konfiguriert ist, dass er die Dehnungssensordaten und Bilddaten mit zweiten Dehnungssensordaten und zweiten Bilddaten, die einem zweiten Benutzer zugeordnet sind, aggregiert, um das Populationsprofil zu generieren (218).

10. Verfahren, umfassend:
Erstellen eines Benutzerprofils (212) für einen Benutzer basierend auf Dehnungssensordaten, die über einen oder mehrere dem Benutzer zugeordnete Dehnungssensoren (106, 107) erfasst werden, wobei das Benutzerprofil die Dehnungssensordaten, historische Sensordaten für den Benutzer und Benutzerparameterdaten einschließt;
Aggregieren der Dehnungsmessdaten für den Benutzer mit Dehnungsmessdaten für eine Benutzerpopulation zum Erstellen eines Populationsprofils (218), das Populationsdaten umfasst;
Bestimmen der Wahrscheinlichkeit, dass der Benutzer (102) ein muskoloskelettalen Dehnungsereignis an einem Körperteil des Benutzers (102) erleidet, auf Basis der Dehnungssensordaten (202) und durch Vergleichen der im Benutzerprofil (212) gespeicherten Benutzerparameterdaten mit dem Populationsprofil (218);
Vorhersagen, dass der Benutzer einem Risiko eines muskoloskelettalen Dehnungsereignis ausgesetzt ist (242), wenn die Dehnungssensordaten (201) darauf hinweisen, dass der Körperteil des Benutzers (102) einer Dehnung ausgesetzt ist, die einen Wert überschreitet, der mit einer durchschnittlichen oder optimalen Belastung des Benutzers auf Basis des Vergleichs der Benutzerparameterdaten mit den Populationsdaten verbunden ist;
und
als Reaktion auf die Vorhersage des muskoloskelettalen Dehnungsereignisses, Übertragen einer Anweisung, die eine Warnung und ein ergonomisches Formmaß (246) einschließt, das von einer Ausgabevorrichtung (112) ausgegeben werden soll, wobei das ergonomische Formmaß auf Basis des Benutzerprofils generiert wird.

11. Verfahren nach Anspruch 10, wobei die Benutzerparameterdaten ein oder mehrere Merkmale des Benutzers umfassen, einschließlich mindestens eines der folgenden: Alter, Geschlecht, Rasse, Gewicht, sportliche Fähigkeiten und etwaige medizinische Zustände.

12. Verfahren nach Anspruch 10 oder 11, wobei die Vorhersage des muskoloskelettalen Dehnungsereignisses auf Biosensordaten (201) basiert, die über einen oder mehrere dem Benutzer zugeordnete Biosensoren (104) erfasst werden.

13. Verfahren nach einem der Ansprüche 10-12, weiter einschließend:
Generieren des ergonomischen Formmaßes auf Basis einer Regel (248), die einen Schwellenwert definiert, der mit der Bewegung des Benutzers verbunden ist.

14. Verfahren nach einem der Ansprüche 10-13, wobei die Vorhersage des muskoloskelettalen Dehnungsereignisses die Ausführung eines neuronalen Netzwerkmodells (238) einschließt.

15. Computerprogramm, umfassend Computerprogrammanweisungen, die, wenn sie von einem Computerprozessor ausgeführt werden, den Computerprozessor veranlassen, das Verfahren nach einem der Ansprüche 10-14 auszuführen, oder ein nichtflüchtiges computerlesbares Medium, das darauf gespeichert ein solches Computerprogramm aufweist.

## Revendications

1. Appareil comprenant :
un générateur de profil utilisateur (208) configuré pour générer un profil utilisateur (212) pour un utilisateur basé sur des données de capteur de contrainte (202) collectées par l'intermédiaire d'un ou de plusieurs capteurs de contrainte (106, 107) associés à l'utilisateur, le profil utilisateur incluant les données de capteur de contrainte, données historiques de capteur pour l'utilisateur et données de paramètres utilisateur;
un agrégateur (114) configuré pour agréger les données de capteur de contrainte pour l'utilisateur (102) avec des données de capteur de contrainte pour une population d'utilisateurs pour générer un profil de population (218) comprenant des données de population ;
un analyseur de performance (224) configuré pour :
déterminer une probabilité que l'utilisateur (102) subit un événement de contrainte musculosquelettique au niveau d'une portion du corps de l'utilisateur (102) sur la base des données de capteur de contrainte (202) et en comparant les données de paramètres utilisateur stockées dans le profil utilisateur (212) au profil de population (218), dans lequel l'analyseur de performance (224) prédit que l'utilisateur risque de subir un événement de contrainte musculosquelettique (242) si les données de capteur de contrainte (201) indiquent que la portion du corps de l'utilisateur (102) est soumise à une contrainte qui dépasse un niveau associé à un effort moyen ou optimal de l'utilisateur sur la base de la comparaison des données de paramètres utilisateur et des données de population ; et
transmettre, en réponse à la prédiction de l'événement de contrainte musculosquelettique, une instruction incluant une alerte à émettre par un dispositif de sortie (112) ; et
un générateur de recommandation de forme ergonomique (244) configuré pour transmettre, en réponse à la prédiction de l'événement de contrainte musculosquelettique, une instruction incluant une mesure de forme ergonomique (246) à émettre par le dispositif de sortie, dans lequel la mesure de forme ergonomique est générée sur la base du profil utilisateur.

2. Appareil selon la revendication 1, dans lequel les données de paramètres utilisateur comprennent une ou plusieurs caractéristiques de l'utilisateur incluant au moins un parmi : âge, genre, ethnicité, poids, capacité athlétique et pathologies.

3. Appareil selon la revendication 1 ou 2, dans lequel l'analyseur de performance est configuré pour prédire l'événement de contrainte musculosquelettique sur la base de données de biocapteur (201) collectées par l'intermédiaire d'un ou de plusieurs biocapteurs (104) associés à l'utilisateur.

4. Appareil selon l'une quelconque des revendications 1-3, dans lequel : le générateur de recommandation de forme ergonomique est configuré pour générer en outre la mesure de forme ergonomique sur la base d'une règle (248) définissant un seuil associé au mouvement de l'utilisateur.

5. Appareil selon l'une quelconque des revendications 1-4, dans lequel l'analyseur de performance est configuré pour exécuter un modèle de réseau neuronal (238) pour prédire l'événement de contrainte musculosquelettique.

6. Appareil selon l'une quelconque des revendications 1-5, dans lequel l'alerte inclut une ou plusieurs parmi une alerte visuelle, une alerte audio ou une alerte de retour haptique.

7. Appareil selon l'une quelconque des revendications 1-6, dans lequel au moins un parmi l'un ou les plusieurs capteurs de contrainte est porté par un tissu portable (107).

8. Appareil selon l'une quelconque des revendications 1-7, incluant un capteur supplémentaire (108), le capteur supplémentaire incluant une caméra (108) configurée pour capturer des données d'image (204) de l'utilisateur dans un environnement (103), et dans lequel l'analyseur de performance est configuré :
pour mettre à jour le profil utilisateur (212) de l'utilisateur sur la base d'une ou de plusieurs données de capteur de contrainte ou des données d'image (204).

9. Appareil selon une quelconque revendication précédente, dans lequel l'utilisateur est un premier utilisateur et le dispositif de commande de forme ergonomique est configuré pour transmettre les données de capteur de contrainte et les données d'image à l'agrégateur, l'agrégateur étant configuré pour agréger les données de capteur de contrainte et les données d'image avec de secondes données de capteur de contrainte et de secondes données d'image associées à un second utilisateur pour générer le profil de population (218).

10. Procédé comprenant :
la génération d'un profil utilisateur (212) pour un utilisateur basé sur des données de capteur de contrainte collectées par l'intermédiaire d'un ou de plusieurs capteurs de contrainte (106, 107) associés à l'utilisateur, le profil utilisateur incluant les données de capteur de contrainte, données historiques de capteur pour l'utilisateur et données de paramètres utilisateur ;
l'agrégation des données de capteur de contrainte pour l'utilisateur avec des données de capteur de contrainte pour une population d'utilisateurs pour générer un profil de population (218) comprenant des données de population ;
la détermination d'une probabilité que l'utilisateur (102) subit un événement de contrainte musculosquelettique au niveau d'une portion du corps de l'utilisateur (102) sur la base des données de capteur de contrainte (202) et en comparant les données de paramètres utilisateur stockées dans le profil utilisateur (212) au profil de population (218) ;
la prédiction que l'utilisateur risque de subir un événement de contrainte musculosquelettique (242) si les données de capteur de contrainte (201) indiquent que la portion du corps de l'utilisateur (102) est soumise à une contrainte qui dépasse un niveau associé à un effort moyen ou optimal de l'utilisateur sur la base de la comparaison des données de paramètres utilisateur et des données de population ;
et
la transmission, en réponse à la prédiction de l'événement de contrainte musculosquelettique, d'une instruction incluant une alerte et une mesure de forme ergonomique (246) à émettre par un dispositif de sortie (112), dans lequel la mesure de forme ergonomique est générée sur la base du profil utilisateur.

11. Procédé selon la revendication 10, dans lequel les données de paramètres utilisateur comprennent une ou plusieurs
caractéristiques de l'utilisateur incluant au moins un parmi : âge, genre, ethnicité, poids, capacité athlétique et pathologies.

12. Procédé selon la revendication 10 ou 11, dans lequel la prédiction de l'événement de contrainte musculosquelettique est basée sur des données de biocapteur (201) collectées par l'intermédiaire d'un ou de plusieurs biocapteurs (104) associés à l'utilisateur.

13. Procédé selon l'une quelconque des revendications 10-12, incluant en outre :
la génération de la mesure de forme ergonomique sur la base d'une règle (248) définissant un seuil associé à un mouvement de l'utilisateur.

14. Procédé selon l'une quelconque des revendications 10-13, dans lequel la prédiction de l'événement de contrainte musculosquelettique inclut l'exécution d'un modèle de réseau neuronal (238).

15. Programme informatique comprenant des instructions de programme informatique qui, lorsqu'elles sont exécutées par un processeur informatique, amènent le processeur informatique à exécuter le procédé selon l'une quelconque des revendications 10-14, ou un support non transitoire lisible par ordinateur sur lequel est stocké un tel programme informatique.
